# EUROPEAN PATENT SPECIFICATION

(11) **EP 4 041 742 B1**
(45) Date of publication and mention of the grant of the patent: **18.10.2023**
(21) Application number: 20780299.2
(22) Date of filing: 18.09.2020
(51) Int. Cl.: C07H 19/00, C12Q 1/6806, C12Q 1/6869

(54) **EPIGENETIC PROFILING METHOD**
EPIGENETISCHPROFILIERUNGS VERFAHREN
METHODS DE PROFILATION EPIGENETIQUE

(30) Priority: 20.09.2019 GB 201913594
(43) Date of publication of application: 17.08.2022
(73) Proprietor: The University of Birmingham, Birmingham, West Midlands B15 2TT (GB)
(72) Inventor: NEELY, Robert, Birmingham, West Midlands B15 2TT (GB); FERNANDEZ-TRILLO, Francisco, Birmingham, West Midlands B15 2TT (GB); KENNEFICK, Jack, Birmingham, West Midlands B15 2TT (GB); WILKINSON, Andrew, Birmingham, West Midlands B15 2TT (GB)
(74) Representative: HGF
(86) International application number: PCT/GB2020/052263
(87) International publication number: WO 2021/053346

(56) References cited:
- EP-A1- 2 594 651
- EDITA KRIUKIENE ET AL: "DNA unmethylome profiling by covalent capture of CpG sites", NATURE COMMUNICATIONS, vol. 4, no. 1, 23 July 2013 (2013-07-23), page 2190, XP055746376, DOI: 10.1038/ncomms3190

## Description

The invention relates to a method for epigenetic profiling. More particularly, the invention relates to a method for epigenetic profiling which comprises selectively functionalizing non-methylated CpG sites of DNA with a linker comprising a hydrolysable moiety.

Epigenetics is the study of heritable modifications to the genome that do not involve an alteration in the DNA sequence. Such modifications include DNA methylation, histone modification and non-coding RNA-associated gene silencing. Epigenetic modifications play crucial roles within the cell, most notably in the regulation of gene expression in eukaryotes. DNA methylation occurs via the addition of a methyl (CH₃) group to DNA. The most widely characterised DNA methylation process is the addition of a methyl group to the 5-carbon of the cytosine ring, resulting in 5-methyl cytosine (5mC). In somatic cells, 5-methyl cytosine is largely restricted to CpG (cytosine-phosphate-guanine) sites (also referred to as "CG" sites). When a CpG site in the promoter region of a gene is methylated, expression of the gene is repressed. The addition of methyl groups to DNA is carried out by enzymes called DNA methyl transferases.

Changes to the organisation of epigenetic modifications have been associated with a growing number of diseases. In recent years there has been considerable interest in research to investigate the role of epigenetic modifications in various diseases including cancer and heart disease, as well as in the mechanisms of ageing and human development.

There are a number of methods available to study the epigenome. However, some of the current approaches suffer from uneven coverage, lack of specificity, harsh chemical treatment to the DNA and/or high cost.

For example EDITA KRIUKIENE ET AL ("DNA unmethylome profiling by covalent capture of CpG sites",NATURE COMMUNICATIONS, vol. 4, no. 1, 2013 article number 2190) describes a method for epigenetic profiling a DNA by labelling the unmethylated CpG group using methyltransferase, separating the labelled fragments using biotin and cleaving the label from the fragment using DTT as reagent.

The present invention has been devised with these issues in mind.

According to a first aspect of the present invention there is provided a method for analyzing DNA, e.g. for epigenetic profiling, the method comprising:
- forming labelled DNA fragments by:
   (a) cleaving genomic DNA into DNA fragments;
   (b) selectively functionalizing any non-methylated CpG sites present in the DNA with a linker comprising a hydrolysable moiety; and
   (c) attaching a label to the linker;
- separating the labelled DNA fragments from any non-labelled DNA fragments;
- hydrolysing the hydrolysable moiety of the linker of the separated labelled DNA fragments, so as to release the DNA fragments from the label;
   and
- sequencing the released DNA fragments.

It will be understood that steps (a), (b) and (c) above may be carried out in any order. For example, the label may be attached to the linker before the DNA is functionalized with the linker. The DNA may be functionalized with the linker (to which the label may or may not be already attached) prior to cleaving the DNA, or after cleaving the DNA. Thus, it will be appreciated that step (b) may be carried out on genomic DNA or on DNA fragments.

In some embodiments step (c) is carried out before step (b). In some embodiments, step (a) is carried out after step (b) or after step (c). In some embodiments, the method is carried out in the order stated, i.e. step (a) followed by step (b) followed by step (c).

In some embodiments, the step of forming the labelled DNA fragments is carried out by performing steps (a), (b) and (c) in the order stated. Thus, in some embodiments, the method of the invention comprises:
- cleaving genomic DNA into DNA fragments;
- selectively functionalizing any non-methylated CpG sites present in the DNA fragments with a linker comprising a hydrolysable moiety, thereby forming functionalized DNA fragments;
- attaching a label to the linker of the functionalized DNA fragments, thereby forming labelled DNA fragments;
- separating the labelled DNA fragments from any non-labelled DNA fragments;
- hydrolysing the hydrolysable moiety of the linker of the separated labelled DNA fragments, so as to release the DNA fragments from the label; and
- sequencing the released DNA fragments.

The genomic DNA may be present in a sample. The sample may have been obtained from an animal, such as a human. The sample may further comprise a suitable diluent or buffer.

Cleavage of genomic DNA may be carried out enzymatically, for example using a restriction enzyme. Alternatively, genomic DNA may be cleaved into fragments using mechanical techniques such as sonication or shearing. In some embodiments, cleavage is carried out using a restriction enzyme that produces sticky ends (i.e. an overhang or stretch of unpaired nucleotides at the end of the resulting DNA fragment). Alternatively, a restriction enzyme may be used which results in DNA fragments with blunt ends (wherein both strands of the resulting DNA fragment terminate in a base pair). Any restriction enzyme may be used which is not sensitive to CpG methylation. An example of a suitable restriction enzyme which results in sticky ends and is non-sensitive to CpG methylation is SaqAl, which cuts DNA at the recognition site T^TAA.

Optionally, the method further comprises the step(s) of isolating the genomic DNA, prior to cleavage. The genomic DNA may be isolated from cells which have been cultured. Protocols for isolating genomic DNA from cells will be known by those skilled in the art. Isolation of genomic DNA may be carried out using a commercially available kit (e.g. as sold by Qiagen), in accordance with the manufacturer's instructions.

In some embodiments the hydrolysable moiety comprises a Schiff base. In some embodiments the Schiff base is a N-substituted hydrozone or an O-substituted oxime.

In some embodiments the hydrolysable moiety comprises a disulphide (S-S) bond.

The linker may have the following general formula: 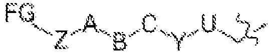
wherein FG represents a functional group comprising a reactive centre;
Z represents a non-reactive group selected from one of an aliphatic linkage or an aromatic linkage;
A-B-C represent the hydrolysable moiety (e.g. the Schiff base moiety);
Y represents a non-reactive group selected from an aliphatic linkage or an aromatic linkage; and
U represents an unsaturated bond selected from one of an alkene, an alkyne, an aryl group, a carbonyl group or a group comprising one or two S=O bonds.

In some embodiments the hydrolysable moiety comprises one of the following structures: 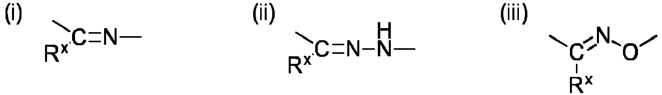 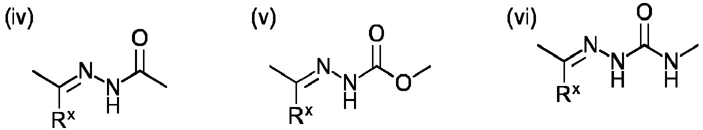 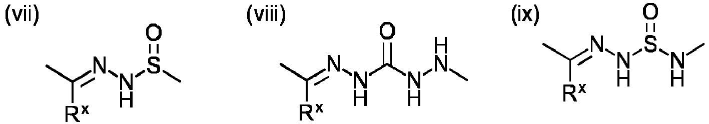 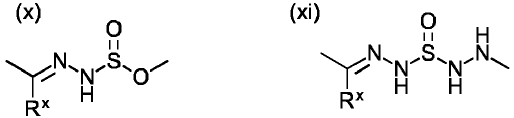 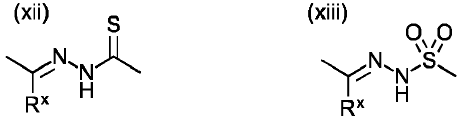 wherein R^{x} represents one of a hydrogen atom, a deuterium atom, an aromatic group or an aliphatic group.

The step of selectively functionalizing any non-methylated CpG sites present in the DNA may comprise forming a covalent bond between the non-methylated CpG sites and the reactive centre of the functional group of the linker.

In some embodiments, the covalent bond formed between the DNA and the reactive centre may be, for example, a carbon-carbon bond, a carbon-nitrogen bond, a carbon-sulphur bond, or a carbon-oxygen bond. In some embodiments, the covalent bond is a carbon-carbon bond.

The covalent bond may be formed between the reactive centre and position 5 of the cytosine ring of the non-methylated CpG site.

Methyltransferases (MTases) are emerging as important tools for the site-selective modification of DNA, RNA, and proteins. In nature, the methyltransferase enzyme catalyses the highly specific transfer of a methyl group from a S-adenosyl-L-methionine cofactor to DNA or RNA. The introduction of methyl groups to these classes of biomolecules helps to regulate gene expression levels within cells.

In mTAG labelling, a S-adenosyl-L-methionine cofactor analogue is employed wherein the methyl group of the natural S-adenosyl-L-methionine cofactor is exchanged for a different moiety, e.g. a linker moiety. A methyltransferase enzyme may then be used to functionalize a target biomolecule with the different moiety using the modified cofactor. By manipulating the chemical structure of the naturally occurring S-adenosyl-L-methionine cofactor, it is possible to use this labelling process as a method for the covalent introduction of functional groups to biomolecules. The linker moiety may comprise further functionality, which may be usable to further modify the biomolecule, e.g. with a label, tag, or a further biomolecule. One of the most common applications explored using this methodology sees the introduction of clickable groups to DNA for the introduction of fluorophores for mapping.

Thus, in some embodiments, the step of selectively functionalizing any non-methylated CpG sites in the DNA (either the genomic DNA or the DNA fragments) with the linker may be carried out using a DNA methyltransferase enzyme. In other words, the method of the invention may comprise mTAG labelling. The methyl transferase may be capable of selectively transferring a transferable group from a S-adenosyl-L-methionine (AdoMet) cofactor analogue to the non-methylated CpG sites of the DNA. In such embodiments, the transferrable group of the S-adenosyl-L-methionine cofactor analogue constitutes the linker with which the DNA is functionalized.

As is known in the art, cytosine-5 methyltransferases can be engineered to direct transfer of extended groups from AdoMet analogues onto CpG sites in DNA. Thus, in some embodiments, the DNA is functionalized using a methyl transferase that displays sensitivity towards CpG methylation. In other words, the methyl transferase specifically methylates CpG sites. The methyl transferase may be a cytosine-5 methyl transferase. Suitable enzymes include M.Hhal, M.Sssl, M.Mpel, M.Taql, and mutants thereof. M.Mpel can be obtained using the methods described by Wojciechowski et al., Proc Natl Acad Sci USA. 2013 Jan 2; 110(1): 105-110. Preparation of M.Sssl is described by Darii et al., Molecular Biology 41, 110-117 (2007). Purification of M. Hhal is described by Kumar et al, Biochemistry (1992), 31 (36), 8648-8653. Preparation of M.Taql is described by Hlz et al., Nucleic Acids Res. 26, 1076-1083 (1998).

In some embodiments the DNA is functionalized using the CpG-specific cytosine-5 methyl transferase M.Mpel. In some embodiments, the methyl transferase is a double mutant (Q136A/N374A) of M.Mpel. These mutations facilitate the use of AdoMet analogues, such as those described herein, by the enzyme. The skilled person would be capable of engineering further cytosine-5 methyltransferases for site-specific labelling of DNA, using standard molecular biology techniques and the teachings of Lukinavicius et al., Nucleic Acids Research, 40, 22 (2012) pages 11594-11602.

In some embodiments, the S-adenosyl-L-methionine (AdoMet) analogue has the following general formula: 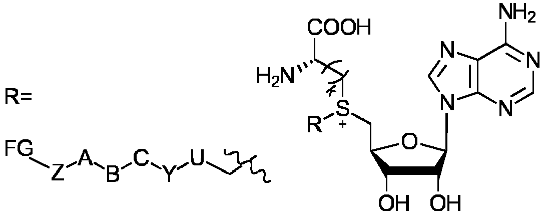
wherein R represents a transferable group (i.e. the linker);
FG represents a functional group;
Z represents a non-reactive group selected from one of an aliphatic linkage or an aromatic linkage;
A-B-C represent the hydrolysable moiety;
Y represents a non-reactive group selected from one of an aliphatic linkage or an aromatic linkage;
U represents an unsaturated bond selected from one of an alkene, an alkyne, an aryl group, a carbonyl group, or a group comprising one or two S=O bonds; and
k represents an integer of 1 or 2.

FG may be selected from one of an azide, an alkyne, an isothiocyanate, or an isocyanate moiety. In some embodiments FG is an azide moiety.

The AdoMet analogue may be associated with a counter ion, which may be one or more of a carbonate anion (CO₃²⁻), a hydrogencarbonate (HCO₃⁻), a tetrafluoroborate anion (BF₄⁻), a hexafluorophosphate anion (PF₆⁻), an acetate (OAc⁻), a trifluoroacetate anion, a formate anion, halide (e.g. F⁻, Cl⁻, Br, I⁻), or a sulphonate anion.

Y may represent a non-reactive aliphatic or aromatic linkage comprising from 1 to 15 atoms in the backbone of the linker, e.g. from 2 to 10 or from 3 to 5 atoms. In some embodiments, Y represents a non-reactive aliphatic or aromatic linkage comprising from 1 to 15 CH₂ moieties, e.g. from 2 to 10 or from 3 to 5 CH₂ moieties.

In some embodiments, Y is an alkyl linkage.

In some embodiments, Z comprises a polyether chain, optionally a polyethylene glycol chain comprising up to 5 monomers of ethylene glycol. Additionally or alternatively, Z may comprise an aromatic group, e.g. a C₆H₄(C=O)NH group.

The method of the invention using the linker molecules may be described as bio-orthogonal.

The unsaturated moiety U, at a β-position to the trivalent sulphonium centre, is believed to enhance the reactivity of the linker to the alkylation of a polynucleotide biomolecule using a methyl transferase enzyme.

In some embodiments, U may represent an alkyne. Thus, in some embodiments, the AdoMet analogue has the following general formula:

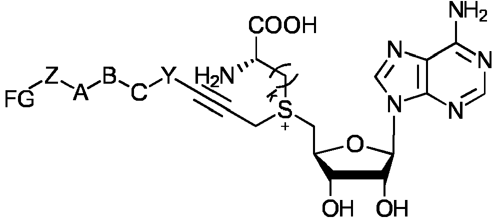

In alternative embodiments, U may represent an alkene. In embodiments, the AdoMet analogue may have the following general formula:

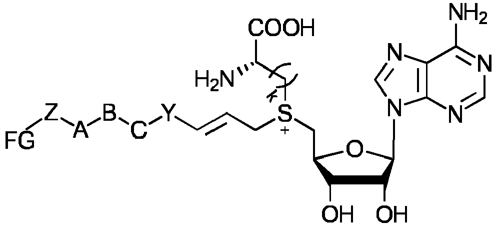

In some embodiments, the AdoMet analogue has the following general formula: 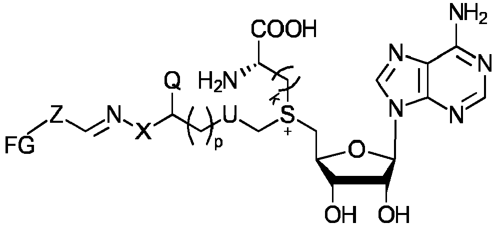
wherein the hydrolysable moiety is a Schiff base moiety comprising the C=N-X-C-Q moiety;
p represents an integer of from 1 to 15, e.g. 1, 2, 3, 4, 5, 6, 7, 8, 9, 10, 11, 12, 13, 14, or 15; or an integer of from 2 to 10; or an integer of from 3 to 5; e.g. p may be 4;
Q represents one of an oxygen atom or two hydrogen atoms independently bonded to the carbon centre;
U represents an unsaturated bond selected from one of an alkene, an alkyne, an aryl group, a carbon atom comprising a carbonyl group, a sulphur atom comprising one or two S=O bonds;
X represents one of an oxygen atom or a nitrogen atom;
Z represents a non-reactive group selected from one of an aliphatic linkage or an aromatic linkage;
FG represents the functional group, which may for example be selected from one of an azide, an alkyne, an isothiocyanate, or an isocyanate moiety; and
k represents an integer of 1 or 2.

In some embodiments, the AdoMet analogue has the following general formula: 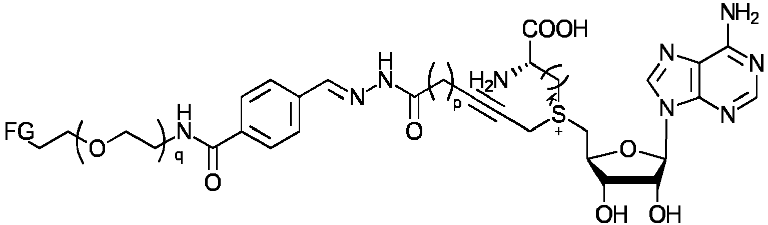
wherein the hydrolysable moiety is a Schiff base moiety comprising the -C=N-N-C=O bond;
p represents an integer of from 1 to 15, e.g. 1, 2, 3, 4, 5, 6, 7, 8, 9, 10, 11, 12, 13, 14, or 15; or an integer of from 2 to 10; or an integer of from 3 to 5; e.g. p may be 4;
q represents an integer of from 1 to 15, e.g. 1, 2, 3, 4, 5, 6, 7, 8, 9, 10, 11, 12, 13, 14, or 15; or an integer of from 2 to 10; or an integer of from 3 to 5, e.g. q may represent 2 or 3;
k represents an integer of 1 or 2; and
FG represents the functional group, which may for example be selected from one of an azide, an alkyne, an isothiocyanate, or an isocyanate moiety.

In some embodiments, the AdoMet analogue has the following general formula: 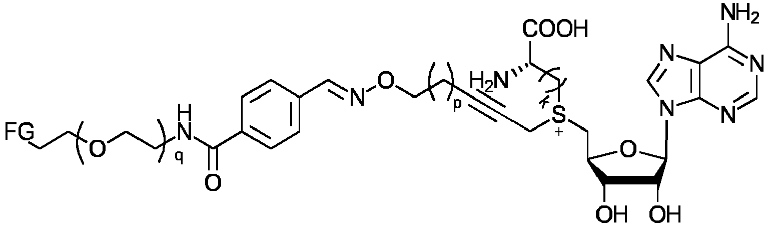
wherein the hydrolysable moiety is a Schiff base moiety comprising the -C=N-O- bond;
p represents an integer of from 1 to 15, e.g. 1, 2, 3, 4, 5, 6, 7, 8, 9, 10, 11, 12, 13, 14, or 15; or an integer of from 2 to 10; or an integer of from 3 to 5; e.g. p may be 4;
wherein q represents an integer of from 1 to 15, e.g. 1, 2, 3, 4, 5, 6, 7, 8, 9, 10, 11, 12, 13, 14, or 15; or an integer of from 2 to 10; or an integer of from 1 to 5, e.g. q may be 2 or 3;
k represents an integer of 1 or 2; and
FG represents the functional group, which may for example be selected from one of an azide, an alkyne, an isothiocyanate, or an isocyanate moiety.

In some embodiments, the AdoMet analogue has the following general formula: 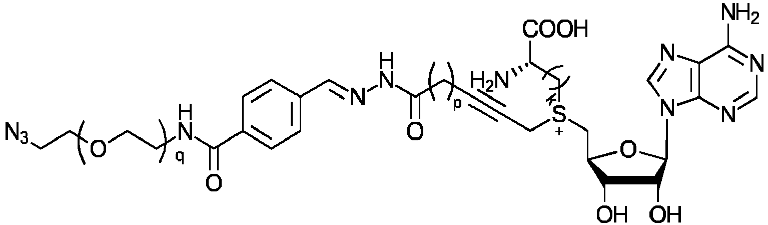 wherein k is 1 or 2, p is 4, and q is 2 or 3.

In some embodiments, the AdoMet analogue has the following general formula: 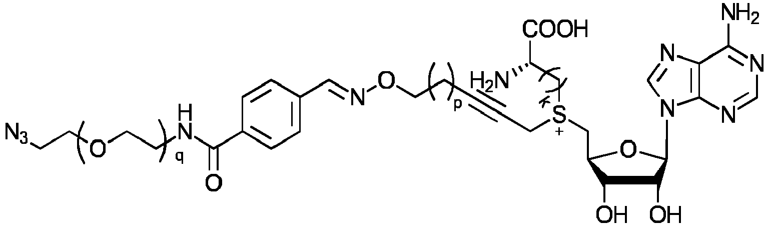 wherein k is 1 or 2, p is 4, and q is 2 or 3.

In any of the above embodiments, k may be 1 or 2. In some embodiments, k is 2.

The step of attaching a label to the linker may comprise forming a covalent bond between the label and the reactive centre of the functional group (FG) of the linker. In some embodiments, the covalent bond formed between the linker and the label may be a carbon-nitrogen bond.

It is to be understood that the functional group (FG) may react with the label by means of a chemical reaction (e.g. click chemistry) or a chemoenzymatic reaction (e.g. in an enzyme-mediated reaction). Any suitable functional group may be selected as the functional group for reaction, which is bio-orthogonal to the hydrolysable moiety A-B-C. For example, suitable reactions involving the functional group and label include click chemistry, cycloadditions, Staudinger reactions, epoxide ring opening reactions, nucleophilic substitutions, and/or nucleophilic additions.

In some embodiments, the functional group may be selected from one of a halide (e.g. an F, Cl, Br, or I atom), an unsaturated bond (e.g. an alkene, an alkyne), an azide, an activated ester, an activated carbonate, a carbamate, an epoxide, an isothiocyanate, or an isocyanate moiety.

In some embodiments, the functional group comprises an azide or an alkyne moiety, for example for reacting with an alkyne or an azide, respectively, on the label.

The label may comprise a second functional group that is capable of reacting with the functional group of the linker molecule to form a covalent bond. Any suitable group may be selected as the second functional group for the reaction, which is bio-orthogonal to the hydrolysable moiety A-B-C. In some embodiments the second functional group is an alkyne or an azide.

The label may comprise any suitable molecule which enables the labelled DNA fragments to be separated from non-labelled DNA fragments. For example, the label may comprise a ligand (such as a protein or antibody) which can be selectively captured by a capture agent (such as a protein receptor or a further antibody).

The skilled person will be aware of many possible types of label/ligand - capture agent interactions which could be utilized in the context of the present invention. These may be broadly categorized by the nature of the interaction. In some embodiments, the interaction between the label (or ligand which forms a part of the label) and a capture agent is a covalent biorthogonal coupling, such as an azide-alkyne, amine-carboxylic acid, sulfhydryl-maleimide, alkoxyamine-aldehyde, or azide-amine coupling. For example, the label may comprise a functional group which is capable of interacting with a functional group of a capture agent so as to form one of the covalent couplings mentioned above.

In some embodiments, the interaction between the label (or ligand) and a capture agent is noncovalent, for example biotin-streptavidin, digoxenin-antidigoxenin, desthiobiotin-streptavidin.

In some embodiments, the label comprises or consists of a protein tag. Suitable protein tags include a CLIP-tag, a SNAP-tag, or a maltose binding protein (which may be captured using amylose agarose).

In some embodiments the label comprises biotin. The use of biotin is advantageous since it enables the selective capture of labelled DNA using streptavidin or avidin as a capture agent.

In some embodiments, the label comprises a ligand conjugated to a moiety comprising the second functional group. For example, the label may comprise biotin conjugated to a chemical moiety comprising an alkyne, for forming a covalent bond with an azide functional group of the linker.

Click reactions between azides and alkynes are often catalyzed by copper(I) catalysts, in a process known as CuAAC (Copper-catalyzed Azide-Alkyne Cycloaddition). An alternative reaction, which avoids the use of copper(I) which is toxic to biological systems, is the strain-promoted azide-alkyne cycloaddition (SPAAC) between an azide and strained alkyne group. Suitable strained alkyne groups include cyclooctyne, MOFO (monofluorinated cyclooctyne), DIFO (difluorinated cyclooctyne), DBCO (dibenzocyclooctyne, also abbreviated to DIBO) and SARAC (biarylazacyclooctynone).

Thus, in some embodiments the second functional group of the label comprises a strained alkyne group such as DBCO. The label may further comprise a ligand, such as biotin, conjugated to the strained alkyne group.

Examples of suitable biotin-DBCO conjugates include the following structures:

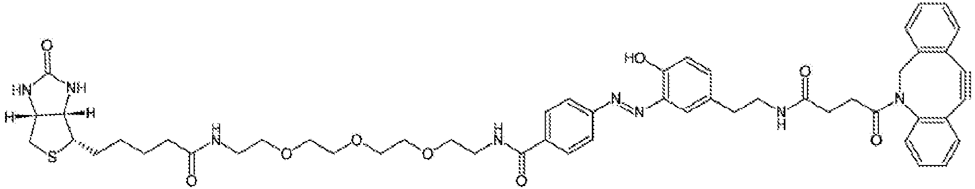

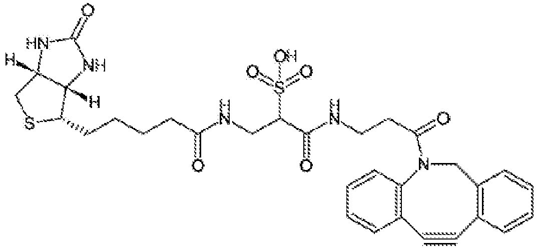

Separating the labelled DNA fragments from any non-labelled DNA fragments may comprise using a capture agent which selectively binds to the label of the labelled DNA fragments. The capture agent does not bind to unlabelled DNA fragments. Since only the DNA fragments comprising non-methylated CpG are labelled, the method of the invention enables the isolation of non-methylated DNA sequences present in genomic DNA.

It will therefore be appreciated that the capture agent will be selected by the skilled person according to the type of label used. For example, if the label comprises a protein, an antibody specific for that protein may be selected as the capture agent.

In some embodiments in which the label comprises biotin, the capture agent may comprise avidin or streptavidin.

The capture agent may be immobilized, for example on a solid support such as beads, a column or an array.

In some embodiments, the capture agent comprises or is streptavidin immobilized on magnetic beads (i.e. magnetic beads with streptavidin covalently coupled to the surface). In such embodiments, DNA fragments labelled with a label comprising biotin can be separated from non-labelled DNA by mixing the labelled DNA fragments with the streptavidin-coated beads. The streptavidin capture agent binds to the biotin label, thereby binding the labelled DNA fragments to the beads. The captured DNA fragments are then separated from non-labelled DNA fragments using a magnet. The skilled person will be aware of other ligand-capture agent pairs and methods suitable for the separation of labelled DNA.

Following separation of the labelled DNA fragments, the hydrolysable moiety of the linker is hydrolysed in order to remove the label and release the DNA fragments. Hydrolysis may be carried out by treating the separated and labelled DNA fragments with a hydrolysing agent. It will be appreciated that the hydrolyzing agent will be selected by the skilled person in accordance with the nature of the hydrolysable moiety. In some embodiments, hydrolysis is carried out using a hydrolysing agent such as an acid or a hydroxylamine, and/or by heating. For example, hydrolysis may be carried out by treatment with hydroxylamine in an ammonium acetate buffer solution.

In some embodiments, the method further comprises ligating the released DNA fragments together, prior to sequencing. Ligating the DNA fragments together to produce longer sequences may be advantageous when the DNA is to be sequenced using certain sequencing platforms, such as nanopore sequencing. However, it will be appreciated that different sequencing platforms may be used for which a ligation step is not required.

Ligation of the released DNA fragments may be carried out enzymatically, using a DNA ligase such as T4 DNA ligase. Ligation protocols will be well-known by those skilled in the art, and are described by Sambrook J, Russell DW, Eds. (2012) Molecular Cloning: A Laboratory Manual. 4th ed. Cold Spring Harbor, NY: Cold Spring Harbor Laboratory. Alternatively, ligation may be carried out using the protocol described herein. The ligation step of the method results in sequences of DNA comprised of many fragments. It will be appreciated that the DNA fragments are ligated randomly, such that in the sequences generated the fragments are in a different order to the order they appeared in the original genomic DNA.

Optionally, the DNA sequences are amplified prior to sequencing. Amplification may be carried after ligation. A number of methods for amplifying DNA *in vitro* are well known, including PCR (polymerase chain reaction), rolling circle amplification, and strand displacement.

In some embodiments, the DNA sequences are amplified by PCR. PCR amplification removes the portion of the linker molecule which remains attached to the DNA fragments following hydrolysis, as well as increasing the quantity of DNA available for sequencing.

As is known in the art, PCR comprises subjecting a reaction mixture to a repeated heating and cooling cycle comprising denaturation at a relatively high temperature (e.g. around 95 °C), primer annealing at a relatively low temperature (e.g. 50-65 °C) and chain extension at a moderate temperature (e.g. around 70-80 °C). The reaction mixture comprises the DNA sequence to be amplified (e.g. the ligated DNA sequences), a pair of primers (single-stranded oligonucleotides having a sequence which is complementary to a region of the target DNA or a PCR adapter sequence), deoxyribonucleoside triphosphates (dNTPs), and a DNA polymerase enzyme (e.g. Taq polymerase) in a suitable buffer. PCR protocols are well-known to those skilled in the art and commercial PCR kits are widely available.

In some embodiments, amplification is carried out prior to hydrolysis of the hydrolysable moiety of the linker so as to release the DNA fragments from the label. In some embodiments, the labelled DNA sequences are amplified while bound to a capture agent which is immobilized on a solid support. Surprisingly, the inventors have found that DNA polymerase is capable of processing DNA with biotin labels, even when the biotin labels are not located at the ends of the DNA but towards the middle of the DNA strand.

In some embodiments, prior to amplification, and after optional ligation, the method further comprises adding PCR adapters to the termini of the released DNA fragments or the ligated DNA sequences. As is known in the art, PCR adapters are DNA oligonucleotides comprising a sequence to which PCR primers can bind.

Prior to the addition of PCR adaptors, the DNA fragments, or the ligated DNA sequences, may be modified. For example, the termini of the fragments or sequences may be blunt-ended and, optionally, dA-tailed. The addition of a single adenine advantageously improves the efficiency of ligation to the adaptor sequences, relative to blunt-ended ligation. The sequencing adaptors may be added to the modified termini of the DNA sequences by ligation, for example using T4 DNA ligase. Such techniques will be well-known to the skilled person, and are described herein.

In some embodiments, the method further comprises adding sequencing adapters to the termini of the released DNA fragments or the ligated DNA sequences, optionally following amplification. As is known in the art, sequencing adapters are DNA oligonucleotides of known sequence which are attached to the ends of DNA sequences prior to sequencing. Adaptor sequences may include one or more of: a sequencing primer site (i.e. a binding site for sequencing primers); platform-specific sequences (for example, sequences which enable binding to flow cells); sample indices (short sequences, typically 6-10 bases, which enable multiplexing); and molecular identifiers (unique codes for each molecule within a library). Adaptor sequences are commercially available. Alternatively, an adaptor sequence may be custom-designed for a particular application.

Sequencing may be carried out using any suitable technique, such as next-generation sequencing (NGS), also known as high-throughput sequencing. NGS technologies include Illumina (Solexa) sequencing, Roche 454 sequencing, Massively parallel signature sequencing (MPSS), Polony sequencing, Combinatorial probe anchor synthesis (cPAS), SOLiD sequencing, Ion Torrent semiconductor sequencing, DNA nanoball sequencing, Heliscope single molecule sequencing, Single molecule real time (SMRT) sequencing, nanopore DNA sequencing and Microfluidic Systems.

In some embodiments, sequencing is performed using nanopore DNA sequencing. In this technique, the sequence is determined by measuring the change in current as a DNA strand is passed through a nanopore.

The method of the invention thus provides affinity-based enrichment of fragmented DNA using methyltransferase-directed DNA modification that targets only the unmethylated CpG sites of the genome. Subsequent labelling and capture leads to isolated of the unmethylated fraction of the genome. This fraction may then be re-ligated for efficient sequencing. This method enables faster and more cost-effective epigenetic profiling, compared to direct sequencing of the entire native genome. Furthermore, by functionalizing the DNA fragments with a linker containing a hydrolysable moiety, the label used to isolated the unmethylated fraction can be efficiently removed for downstream processing.

It will be appreciated that any of the embodiments described herein may be combined with any other embodiment, unless otherwise stated.

Embodiments of the invention will now be described by way of example and with reference to the accompanying figures, in which:
Figure 1 is a reaction scheme for the formation of S-adenosyl-L-methionine cofactor analogues;
Figure 2 provides an overview of the method in accordance with an embodiment of the present invention;
Figure 3 shows molecular structures produced by a method in accordance with an embodiment of the present invention;
Figure 4 is a plot showing the efficiency of capture of biotinylated DNA fragments on streptavidin-coated beads, the DNA fragments containing zero, one, two, three or four unmethylated CpG sites;
Figure 5 is a plot showing the efficiency of capture and release of biotinylated DNA fragments using streptavidin-coated beads, the DNA fragments having been generated by cleaving human genomic DNA;
Figure 6 is a graph showing the lengths of DNA sequences generated by ligating and amplifying released DNA fragments;
Figure 7 is a plot of the aligned read lengths against the sequenced read lengths;
Figure 8 is an alignment of sequencing reads of a gene promoter region obtained by the method of the invention (SUURF ID1, 2 and 3) with a sequencing read obtained by MeDIP; and
Figure 9 shows the results of illumina sequencing.

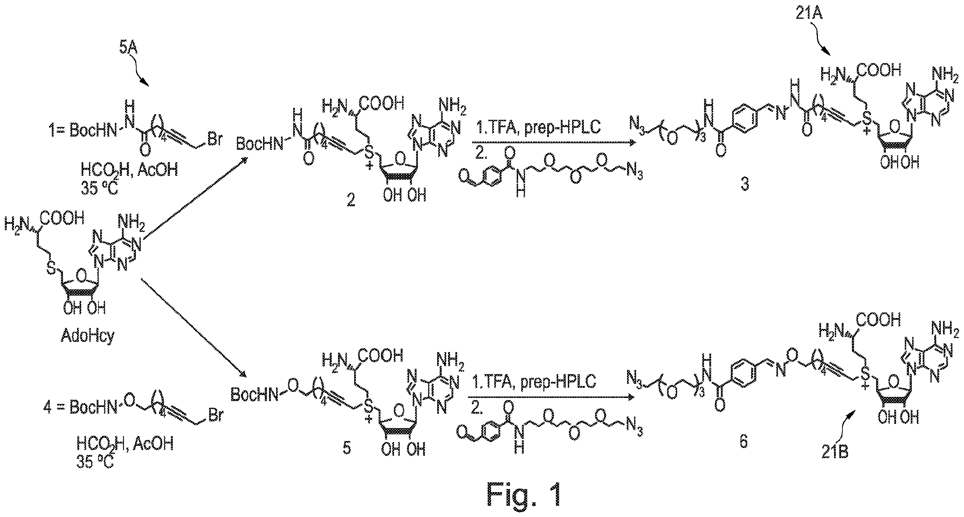

With reference to Figure 1, there is shown a reaction scheme for the formation of S-adenosyl-L-methionine cofactor (AdoMet) analogues.

### Example 1: Synthesis of S-adenosyl-I-methionine Cofactor Analogues

### Synthesis of precursor 1

### Synthesis of 8-hydroxyoct-6-ynoic acid 7.

A solution of 6-heptynoic acid (2 g, 15.87 mmol) was made in dry THF (42 ml) under argon, to this HMPA (34.9 mmol, 6.13 ml) was added and the solution was cooled to -78 °C. To this nBuLi (1.6 M in hexanes, 34.9 mmol, 21.8 ml) was added dropwise whilst maintaining the temperature below -60 °C. The solution was then warmed to -40 °C and stirred for 1 hour. After 1 hour paraformaldehyde (1.47 g, 47.6 mmol) was added via powder funnel under an argon flow. The reaction mixture was then warmed to 45 °C for 4 hours. After reaction, the mixture was quenched with 1 M HCl to pH 4-5 and extracted with EtOAc. The solvent was then dried and the EtOAc was removed by rotary evaporation giving the crude product. Purification was completed using flash column chromatography (silica gel, Hex:EtOAc, 6:4): Yield = 68%, Rf = 0.27 (Hex:EtOAc, 6:4); 1H NMR (300 MHz, DMSO-d6) δ 12.03 (s, 1H), 5.03 (s, 1H), 4.02 (d, J = 2.6 Hz, 2H), 2.29 - 2.14 (m, 4H), 1.63 - 1.50 (m, 2H), 1.50 - 1.39 (m, 2H); MS: m/z [M-H] = 155.46.

### Synthesis of tert-butyl 2-(8-hydroxyoct-6-ynoyl)hydrazine-l-carboxylate 8

8-hydroxyoct-6-ynoic acid **7** (1.35 g, 8.65 mmol) and tert-butyl carbazate (1.4 g, 10.38 mmol) were dissolved in 2:1 THF:H2O (13.5:6.75 ml). To this EDC.HCl (1.87 g, 9.52 mmol) was added slowly over 15 minutes. The mixture was left to stir for 3 hours and then extracted with EtOAc. The organic layer was washed with 0.1 M HCl, water and brine and then the organic layer is collected, dried over anhydrous sodium sulfate and the and the solvent was removed under reduced pressure yielding the product as a white solid: Yield = 63%; 1H NMR (400 MHz, DMSO-d6) δ 9.47 (s, 1H), 8.66 (s, 1H), 5.04 (t, J = 5.9 Hz, 1H), 4.02 (dt, J = 5.9, 2.2 Hz, 2H), 2.19 (tt, J = 7.1, 2.2 Hz, 2H), 2.06 (t, J = 7.2 Hz, 2H), 1.58 (p, J = 7.3 Hz, 2H), 1.50 - 1.32 (m, 12H); 13C NMR (101 MHz, DMSO) δ 172.01, 84.36, 80.94, 79.42, 49.59, 33.06, 28.53, 28.08, 24.70, 18.24; MS: m/z [M+Na] = 294.15.

### Synthesis of tert-butyl 2-(8-bromooct-6-vnovl)hydrazine-1-carboxylate 1

A solution of tert-butyl 2-(8-hydroxyoct-6-ynoyl)hydrazine-1-carboxylate **8** (300 mg, 1.11 mmol) was made in dry DCM (3.33 ml) and cooled on ice. Triphenylphosphine (437 mg, 1.67 mmol) was added and left to dissolve, once dissolved tetrabromomethane (552 mg, 1.67 mmol) was added slowly. The reaction was then brought to room temperature and left to stir for 1 hour. After reaction the solvent was removed under reduced pressure and the crude mixture was purified by flash column chromatography (silica gel Hex:EtOAc, 7:3): Yield = 55%; Rf = 0.15 (Hex:EtOAc 7:3); 1H NMR (300 MHz, DMSO-d6) δ 9.48 (s, 1H), 8.67 (s, 1H), 4.21 (t, J = 2.3 Hz, 2H), 2.27 (tt, J = 6.9, 3.4 Hz, 2H), 2.06 (t, J = 7.4 Hz, 2H), 1.65 - 1.31 (m, 13H); 13C NMR (101 MHz, DMSO) δ 171.4, 155.2, 87.7, 78.9, 76.3, 54.9, 39.5, 32.5, 28.0, 27.3, 24.1, 17.9, 17.2; MS: m/z [M+Na] = 355/357.08.

### Synthesis of Precursor 4

### Synthesis of 7-Bromo-hept-1-yne 9

A solution of 6-heptyn-1-ol (5g, 44.6 mmol) was made in dry DCM (60 ml) and cooled on ice. To this triphenylphosphine (17.6 g, 67 mmol) was added, upon complete dissolution tetrabromomethane (22.2 g, 67 mmol) was added slowly. The reaction mixture was brought to room temperature and stirred for 1 hr. After completion, the solvent was removed under reduced pressure. Hexane was added to the crude forming a white suspension. The hexanefraction was filtered, collected and then the solvent was removed. An oily residue remained which was purified by flash column chromatography with hexane: Yield = 91%, Rf = 0.45 (hexane); %); □max(neat)/cm-1 540 (C-Br); 1H NMR (300 MHz, DMSO-d6) δ 3.53 (t, J = 6.7 Hz, 2H), 2.75 (t, J = 2.7 Hz, 1H), 2.23 - 2.10 (m, 2H), 1.89 - 1.74 (m, 2H), 1.50 - 1.43 (m, 4H).

### Synthesis of 8-bromooct-2-yn-1-ol 10

A solution of 7-bromohept-1-yne **9** (20.56 mmol, 3600 mg) was made in Dry THF (12.3 ml) and cooled to -78 °C under Argon. To this a solution of nBuLi in hexanes (1.6 M, 13 ml) was added dropwise, whilst maintaining the temperature below -60 °C. The reaction mixture was then warmed to 0 °C in an ice bath at which point paraformaldehyde (1718 mg, 55.5 mmol) was added under a flow of Argon and stirred for 30 minutes. The mixture was then warmed to room temperature and left to stir, the temperature was maintained below 30 °C until the exothermic reaction had stopped. The mixture was then heated to 45 °C for 2 hrs. Once complete the reaction was extracted with ether and sat. NH4CI. The organic layer was collected and the solvents were removed under reduced pressure to yield the crude product as an oil. Once dry, purification was completed by flash column chromatography (silica gel, Hexane: Ethyl Acetate, 9:1). The product was then collected as a colourless oil: Yield = 55%, Rf = 0.15 (Hex: EtOAc 9:1), 1H NMR (300 MHz, DMSO-d6) δ 5.04 (t, J = 5.7 Hz, 1H), 4.03 (dt, J = 5.5, 2.1 Hz, 2H), 3.54 (t, J = 6.7 Hz, 2H), 2.20 (m, 2H), 1.88 - 1.75 (m, 2H), 1.52 - 1.40 (m, 4H).

### Synthesis of tert-butyl ((8-hydroxyoct-6-yn-1-yl)oxy)carbamate 11

To a solution of N-Boc Hydroxyl amine (890 mg, 6.55 mmol) in DMF (4.3 ml) 8-bromooct-2- yn-1-ol **10** (1200 mg, 5.85 mmol) and 1,8-Diazabicyclo[5.4.0]undec-7-ene (1000 mg, 6.55 mmol) was added. The solution was stirred at 50 °C for 20 hrs. Once complete, the reaction was extracted with DCM and 15% citric acid solution. The organic phases were dried and collected and the solvent was removed under reduced pressure. A colourless oil was collected as the crude product. This was further purified by flash column chromatography (silica gel, Hexane: Ethyl Acetate, 8:2). The product was collected as a colourless oil: Yield = 73 %, Rf = 0.27; 1H NMR (300 MHz, DMSO-d6) δ 9.91 (s, 1H), 5.03 (t, J = 5.9 Hz, 1H), 4.02 (dt, J = 5.9, 2.2 Hz, 2H), 3.66 (t, J = 6.2 Hz, 2H), 2.17 (tt, J = 6.7, 1.7 Hz, 2H), 1.40 (m, 15H); MS: m/z [M+H] = 258.2.

### Synthesis of tert-butyl ((8-bromooct-6-yn-1-yl)oxy)carbamate 4

A solution of tert-butyl((8-hydroxyoct-6-yn-1-yl)oxy)carbamate **11** (1 g, 3.89 mmol) was made in dry DCM (5.2 ml) and cooled on ice. To this triphenylphosphine (1.53 g, 67 mmol) was added. Upon complete dissolution tetrabromomethane (1.94 g, 67 mmol) was added slowly. The reaction mixture was brought to room temperature and allowed to stir for 1 hr. After completion, the solvent was removed under reduced pressure. Purification was completed using flash column chromatography (silica gel, Hexane: Ethyl Acetate, 8:2): Yield = 67%, Rf 0.52 (Hex:EtOAc, 8:2); λmax(neat)/cm-1 1712 (C=O), 607 (C-Br); 1H NMR (300 MHz, DMSO-d6) δ 9.90 (s, 1H), 4.21 (t, J = 2.4 Hz, 2H), 3.66 (t, J = 6.2 Hz, 2H), 2.25 (tt, J = 6.9, 2.4 Hz, 2H), 1.40 (m, 15H); 13C NMR (101 MHz, DMSO) δ 156.04, 87.85, 79.37, 76.22, 75.05, 39.52, 28.05, 27.64, 27.04, 24.76, 18.06, 17.25; MS: m/z [M+Na] = 342.35/344.35, [M-tBuOH] = 246.38/248.38.

### General coupling procedure

Precursors **1, 4** were reacted with S-adenosyl-L-homocysteine under acidic conditions to give reversible and rewritable Boc-protected AdoMet derivatives.

A solution of S-adenosyl-I-homocysteine (15 mg, 0.04 mmol) was made in a 1:1 mixture of formic and acetic acid (300 µl). Precursor 1 or **4** (tert-butyl 2-(8-bromooct-6-ynoyl)hydrazone-1-carboxylate or tert-butyl ((8-bromooct-6-yn-1-yl)oxy)carbamate) (1.2 mmol, 30 equivs) was then added dropwise, on ice. The reaction mixture was warmed to 35 °C and left to stir overnight. After overnight stirring the reaction mixture was extracted with diethyl ether and the aqueous layer was collected and dried by lyophilisation: MS: m/z [M+H] = 638 (2), [M+H] = 624 (5).

### Cofactor Deprotection

The AdoMet analogues were deprotected under acidic conditions to reveal the hydrazone or alkoxyamine moieties. The crude product was dissolved in TFA (400 µl) and left stir for 2 hrs at room temperature. After reaction the acid was removed under a flow of argon.

### Cofactor Purification

Any excess precursor was removed by purification.

Both diastereomers of the deprotected cofactors could be separated by HPLC, a separation which was not possible at later stages.

The crude reaction mixture was then dissolved in water (2 ml). Purification of AdoMet analogues was performed by preparative reversed-phase HPLC (ACE 5 C-18 25 × 2.12 cm) eluting with 20 mM Ammonium Acetate pH 5.5 Water (A)/MeOH (B) gradient at a flow rate of 10 ml/min. Gradient system: 30 mins 3-30% B, 30-97% B over 30 mins, hold at 97% B for 5 minutes, stop programme. Retention times: Hydrazide iso. 1 = 17.51 mins, iso. 2 = 18.73 mins, hydroxylamine iso. 1 = 25.47 mins, iso. 2 = 28.24 mins: MS: m/z [M+H] = 538 (2), [M+H] = 524 (5).

The deprotected AdoMet derivatives slowly degrade, in particular following freeze-drying, via multiple pathways, giving additional peaks at higher retention times.

### Aldehyde coupling

To mitigate against degradation the AdoMet derivatives were reacted with a commercially available benzaldehyde immediately after purification by HPLC in order to minimise side reactions due to the nucleophilic nature of the hydrazone and alkoxyamine moieties.

To the collected HPLC fractions Ald-PEG3-N3 (1.2 equivs) was added and rolled for 30 mins at room temperature. The fractions were then dried by lyophilsation. Once dry the solids were dissolved in 100 µl 0.1% Acetic Acid and stored at -20 °C. Concentrations were determined by UV absorption analysis with ε260 = 15.400 dm-3 mol-1 cm-1: MS: m/z [M+H] = 867 (3), [M+H] = 856 (6).

The resulting AdoMet analogues contain reactive terminal azides that can be readily conjugated to a range if functional groups, while condensation of the aldehyde with the hydrazone or alkoxyamine incorporates a dynamic functionality, that can be reversibly functionalised.

A slight excess of aldehyde (1.2 equivs) was employed to ensure full functionalisation of the deprotected intermediate.

No degradation of the freeze-dried AdoMet analogues was observed.

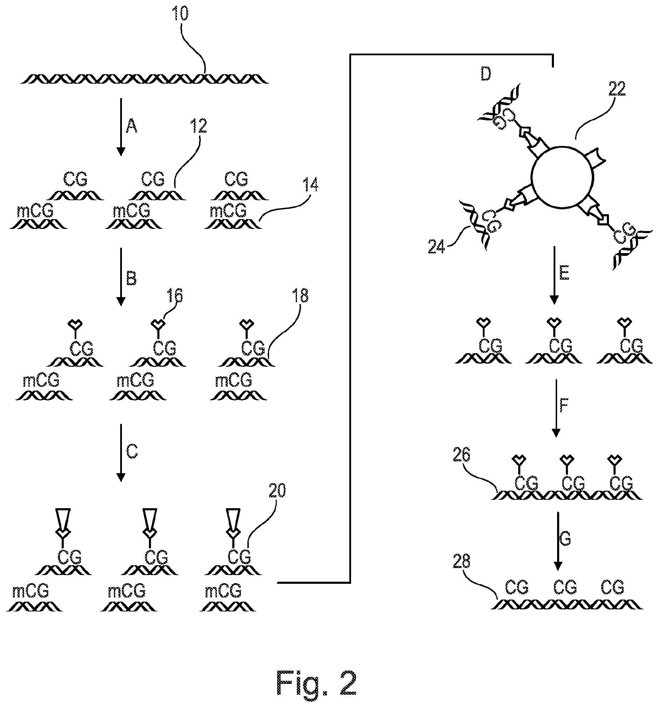

With reference to Figure 2, a method in accordance with the present invention is used for epigenetic profiling of genomic DNA (10), such as human genomic DNA. In a first step (A), the genomic DNA is digested into DNA fragments using a restriction enzyme such as SaqAl. The DNA fragments produced by the enzymatic digestion include fragments which have not been methylated at CpG sites (12) and fragments with have been methylated at CpG sites (14).

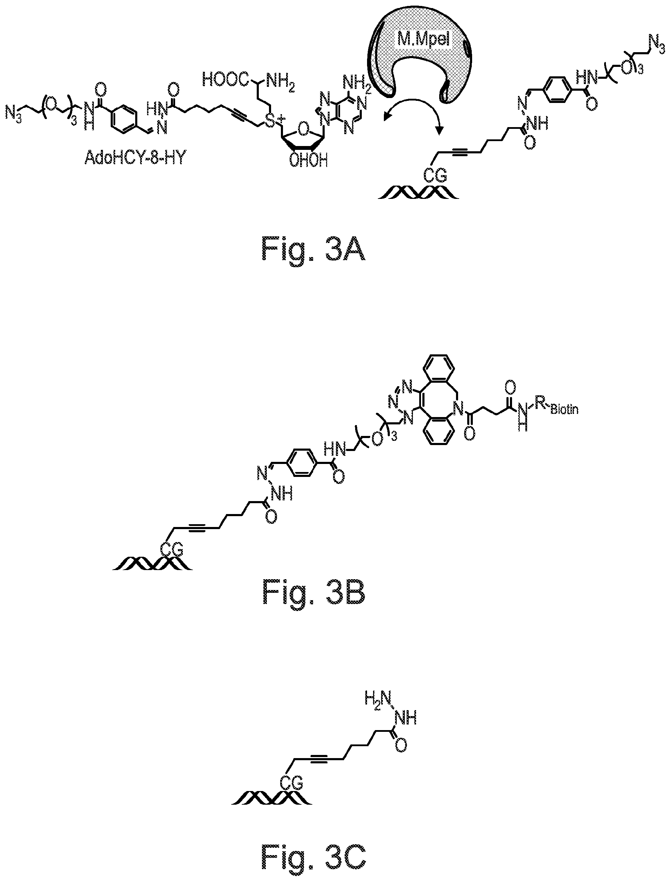

Step (B) comprises methyltransferase directed unmethylated CpG functionalization. In this step, the non-methylated CpG sites present in the DNA fragments (12) are functionalized using a methyl transferase enzyme (such as M.Mpel) and the S-adenosyl-L-methionine (AdoMet) analogue AdoHCY-8-HY (shown in Figure 3A). The methyltransferase transfers a transferable group, i.e. a linker (16) from the cofactor to position 5 of the cytosine of non-methylated CpG sites, thereby producing functionalized DNA fragments (18).

As shown in Figure 3A, the linker which is transferred from the cofactor to the DNA fragments comprises a hydrolysable C=N moiety (a Schiff base), and a terminal azide (N₃) group.

In step (C), the functionalized DNA fragments (18) are reacted with diazo biotin-DBCO, forming labelled DNA fragments (20). A covalent bond is formed between the linker and the biotin label by virtue of a click reaction between the terminal azide of the linker and the alkyne of the DBCO moiety, resulting in the structure shown in Figure 3B.

In step (D), the labelled DNA fragments (20) are captured using streptavidin-coated beads (22). The beads (22) are then washed to remove any non-specifically bound (i.e. non-labelled) DNA. Captured DNA fragments (24) are then released by hydrolyzing the hydrolysable moiety (step (E)), giving the structure shown in Figure 3C.

The released fragments are then re-ligated together in a random fashion using DNA ligase (step (F)). This creates long sequences of DNA (26) comprised of many ligated DNA fragments containing the CpG sites which were not methylated in the original genomic DNA sequence. Optionally, the ligated DNA is amplified by PCR to remove the linkers (step (G)). Finally, the amplified DNA (28) is sequenced.

### Example 2: Capture and release of control DNA fragments that contain varying numbers of capture sites

### Methodology

### PCR amplification of CpG site containing fragments

DNA fragments (~150bp) containing 0, 1, 2, 3 or 4 CpG sites were produced by PCR amplification of sections of the Lambda genome (NEB) using Q5^{®} High-Fidelity DNA Polymerase (NEB) following manufacturer's instructions. The following amplification programme was used: 98 °C for 30 s, 30 cycles 98 °C 10 s, 61 °C 30 s, 72 °C 20 s and a final extension at 72 °C for 2 mins. After amplification the DNA was purified using 2x AMPure XP beads and eluted into 100 µl EB (10 mM Tris-HCl (pH 8.5)). The DNA concentration was quantified using Qubit^{™} 4 Fluorometer using the dsDNA BR Assay Kit (Thermo Fisher) and sized using the High Sensitivity D5000 ScreenTape on the TapeStation 2200 (Agilent).

### CpG capture analysis

750 ng of CpG site (0, 1, 2, 3 or 4) containing PCR fragments were mTAG labelled in 35 µl reactions containing 10x outsmart buffer (3.5 µl)(NEB), 500 µM AdoHcy-8-Hy cofactor (1.17 µl), M.Mpel enzyme (double mutant (Q136A/N374A), 2.5 µl)(1.7 mg/ml) and water. Samples were incubated at 37 °C for 1 hr. Following this 1 µL proteinase K (800 units/ml) (NEB) was added and samples were incubated for 1 hr at 50 °C. Next, 1 µl of Diazo Biotin-DBCO (Jena bioscience) was added and the samples incubated at 37 °C for 1 hr at 1000 rpm. Samples were then purified using 2x AMPure XP beads (washed 2x with 500 µl 80% ethanol) and eluted into Tris buffer A (10 mM Tris, 1 mM Nacl, pH 7.5). The DNA concentration was quantified using Qubit^{™} 4 Fluorometer and dsDNA HS Assay Kit (Thermo Fisher).

5 µl of Dynabeads^{™} MyOne^{™} Streptavidin C1 beads (Thermo Fisher) for each sample were washed 2x with an equal volume of Tris buffer A. The wash solution was removed, and 250 ng of biotin labelled PCR DNA fragments in 5 µL Tris buffer A, were added to the 5 µl of washed streptavidin beads and incubated at RT, 1000rpm for 20 mins. Samples were placed onto a magnet and the supernatant removed and stored. The beads were then washed 2x with 5 µl Tris buffer A and the washes were stored. The percentage of DNA captured was calculated using the Qubit^{™} 4 Fluorometer with the dsDNA HS Assay Kit (Thermo Fisher).

### Results

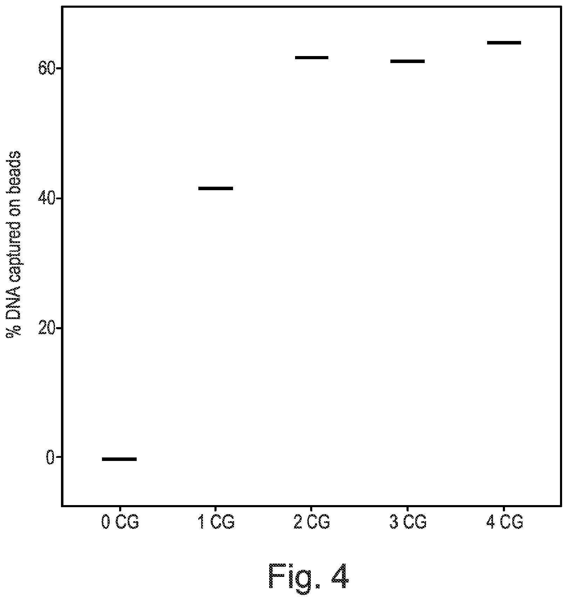

The results of this experiment are shown in Figure 4. This demonstrates the ability to capture control fragments of DNA that contain varying numbers of CpG sites. DNA which did not possess a capture site (0 CG) was not captured, whereas DNA that contained one (1 CG), two (2 CG), three (3 CG) or four (4 CG) capture sites were each captured efficiently. This demonstrates the specificity and efficiency of the method.

### Example 3: Epigenetic study using human DNA

### Methodology

### Genomic DNA extraction

Human genomic DNA was extracted from cultured GM12878 human cells (Coriell: GM12878). Cell culture was done using Epstein-Barr virus (EBV)-transformed B lymphocyte culture from the GM12878 cell line, grown in RPMI-1640 media, supplemented with 2 mM L-glutamine, 15% FBS and incubated at 37 °C. Genomic DNA was extracted using the QIAGEN Genomic-tip 500/G kit (Qiagen) following manufacturer's instructions.

### Digestion of genomic DNA

Human genomic DNA (NA12878) was digested using Anza^{™} 64 SaqAl (Thermo Fisher), in an 80 µL reaction containing 4 µg DNA, 8 µL buffer, 4 µl SaqAl enzyme and water. The reaction was then incubated at 37 °C for 1 hr. The fragmented DNA was cleaned using the QIAquick PCR Purification Kit (Qiagen) and eluted into 30 µl EB (10 mM Tris-HCl (pH 8.5)). If additional DNA was required, the reaction was repeated. The DNA concentration was quantified using the Qubit^{™} 4 Fluorometer and dsDNA HS Assay Kit (Thermo Fisher) and sized using High Sensitivity D5000 ScreenTape on the TapeStation 2200 (Agilent).

### mTAG-directed functionalisation of human DNA and enrichment of unmethylated CpG containing fragments

mTAG labelling and biotin tagging of human DNA fragments was done 3x. Each reaction contained 1.5 µg of SaqAI digested human DNA, 7 µl 10x outsmart buffer, 2.33 µl AdoHcy-8-Hy cofactor (500 µM), 5 µL M.Mpel enzyme (double mutant (Q136A/N374A)) (1.7 mg/ml) and water to a final volume of 70 µl. Samples were incubated at 37 °C for 1 hr. To these reactions 2 µl proteinase K (800 units/ml) (NEB) was added and incubated at 50 °C for 1 hr. Next, 2 µl Diazo Biotin-DBCO (Jena bioscience) was added and reactions were incubated for a further 1 hr at 37 °C, 1000 rpm. Each sample was purified using 2x AMPure XP beads (washed 2x with 1000 µl 80% ethanol) and eluted into 30 µl Tris buffer A (10 mM Tris, 1 mM Nacl, pH 7.5). The DNA concentration was quantified using the Qubit^{™} 4 Fluorometer and dsDNA HS Assay Kit (Thermo Fisher).

20 µl of Dynabeads^{™} MyOne^{™} Streptavidin C1 beads (Thermo Fisher) for each sample were washed 2x with an equal volume of Tris buffer A. 1 µg of labelled and biotin tagged DNA in 30 µl Tris buffer A from each sample was added to 20 µl of washed streptavidin beads and incubated at RT, 1000 rpm for 20 mins. Samples were then placed onto a magnet and the supernatant was removed and beads were washed 2x in 20 µl Tris buffer A. To release the captured DNA from the beads, 80 µl of release buffer (11.2 mM ammonium acetate (pH 6.5), 1M NaCl) along with 20 µl of 0.85 M hydroxylamine solution (170 mM final) was added and incubated at 50 °C, 1000 rpm for 1 hr. Released DNA was then purified using 2x AMPure XP beads and eluted into 14 µl EB (10 mM Tris-HCl (pH 8.5)). The DNA concentration was quantified using the Qubit^{™} 4 Fluorometer with the dsDNA HS Assay Kit (Thermo Fisher) and sized using the High Sensitivity D5000 ScreenTape on the TapeStation 2200 (Agilent).

### Ligation and PCR amplification of released fragments

After quantification and sizing of released human DNA fragments -170-190 ng of DNA remained in each 10.5 µl sample. To this an equal volume (10.5 µl) of Anza^{™} T4 DNA Ligase Master Mix (Thermo Fisher) was added and samples were incubated at RT for 1 hr. Each sample was then purified using 2x AMPure XP beads and eluted into 13 µl EB (10 mM Tris-HCl (pH 8.5)). DNA concentration was quantified using the Qubit^{™} 4 Fluorometer with the dsDNA HS Assay Kit (Thermo Fisher) and sized using the Genomic DNA ScreenTape on the TapeStation 2200 (Agilent).

Following this, the remaining 10 µl of each sample was end-repaired and dA-tailed using NEBNext^{®} Ultra^{™} II End Repair/dA-Tailing Module (NEB). DNA was then cleaned using 2x AMPure XP beads and eluted into 15 µl EB (10 mM Tris-HCl (pH 8.5)). To the 15 µl of end repaired and dA-tailed DNA, 10 µl PCA (Oxford Nanopore Technologies (ONT)) and 25 µl Blunt/TA Ligase Master Mix was added and samples were incubated at 25 °C for 1 hr. The DNA was then purified using 2x AMPure XP beads and eluted into 12 µl EB. The DNA concentration was quantified using the Qubit^{™} 4 Fluorometer with the dsDNA HS Assay Kit (Thermo Fisher).

Next, 3× 50 µl PCR reactions were prepared, containing 4.5 µl (10 ng) PCA ligated DNA from the previous step, 1.5 µl dNTPs (10 mM), 2 µl PRM (Oxford Nanopore Technologies (ONT)), 10 µl LongAmp^{®} Taq reaction buffer (NEB), 2 µl LongAmp^{®} Taq DNA Polymerase (NEB) and 30 µl water. The following amplification programme was used: 94 °C for 2 mins, 21 cycles 94 °C 30 s, 62 °C 15 s, 65 °C 15 mins and a final extension at 65 °C for 15 mins. DNA was purified using 2x AMPure XP beads and the DNA concentration was quantified using the using the Qubit^{™} 4 Fluorometer with the dsDNA BR Assay Kit (Thermo Fisher) and sized using the Genomic DNA ScreenTape on the TapeStation 2200 (Agilent).

### Library preparation and MiniON sequencing

1 µg of re-ligated and PCR amplified DNA in 50 µl of nuclease-free water was end-repaired and dA-tailed using the NEBNext^{®} Ultra^{™} II End Repair/dA-Tailing Module (NEB). Samples were then purified using 2x AMPure XP beads and eluted into Nuclease-free water. Sequencing adapters (AMX) were then ligated using NEBNext Quick T4 DNA Ligase (NEB) following the manufacturer's protocol (1D genomic DNA by ligation (SQK-LSK109) (Oxford Nanopore Technologies (ONT)).

Each library was loaded onto a R9.4.1 flow cell (FLO-MIN106D) following manufacturer's instructions (ONT) and sequenced for 48 hours, using the standard parameters specified for the library preparation protocol. Base-calling was done using Guppy (2.0.10), with parameters based on the library preparation method.

### Read alignment

The sequenced reads were mapped to the human genome reference (hg19) using minimap2¹ with the "-ax map-ont -K 500M" options.

### Results

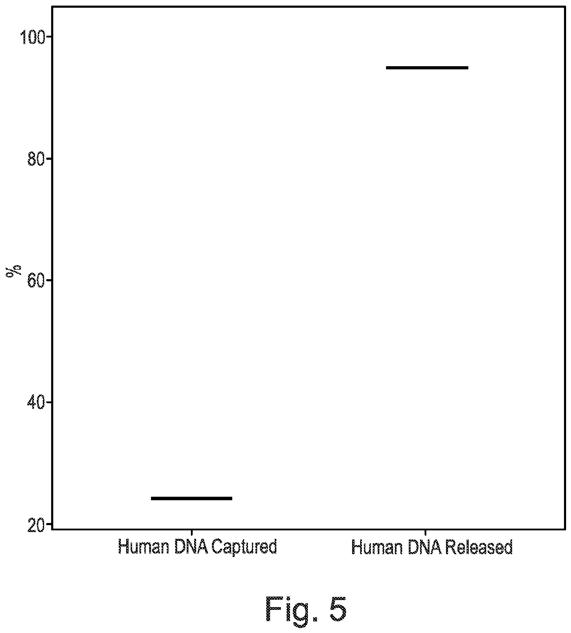

Initial digestion of the human genomic DNA with the SaqAl enzyme was found to yield DNA fragments of about 150bp in length. Following mTAG functionalization of the DNA fragments using the AdoHcy-8-Hy cofactor, and labelling using Diazo Biotin-DBCO, the labelled DNA fragments were captured on streptavidin-coated beads. Consistent capture of about 24% of DNA across all three samples was observed. In addition, highly efficient recovery of the captured DNA from the streptavidin beads (-95%) was achieved in a single step, for all samples (Figure 5).

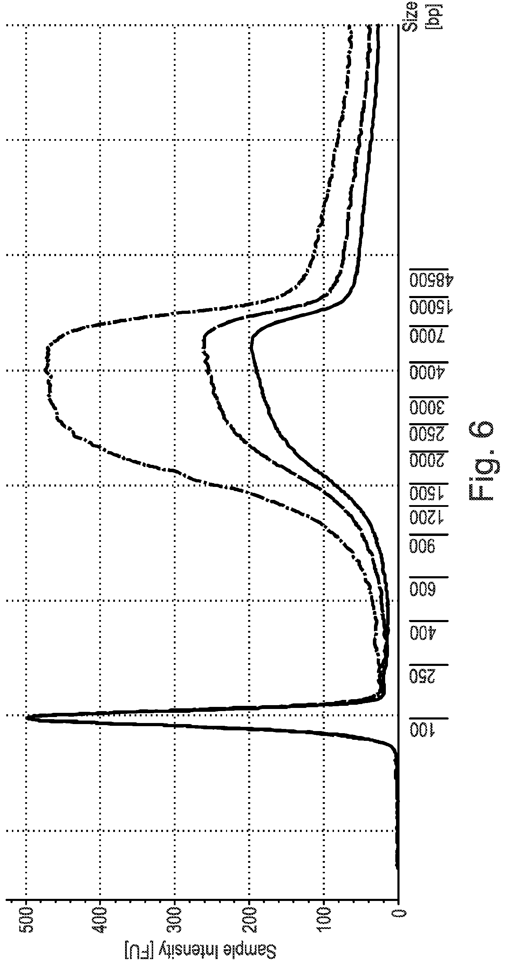

The released fragments of DNA from each sample were then randomly stuck together to form long fragments of DNA. Figure 6 shows the successful re-ligation and PCR amplification of the captured and released DNA fragments in all three repeats of the experiment.

The released and re-ligated human DNA from each sample was then sequenced using a MinilON nanopore sequencing device. As each sequencing read consists of many short fragments of DNA ligated/stuck together randomly, each individual fragment in the read was aligned to the genomic location from which it was derived, using publically available algorithms.

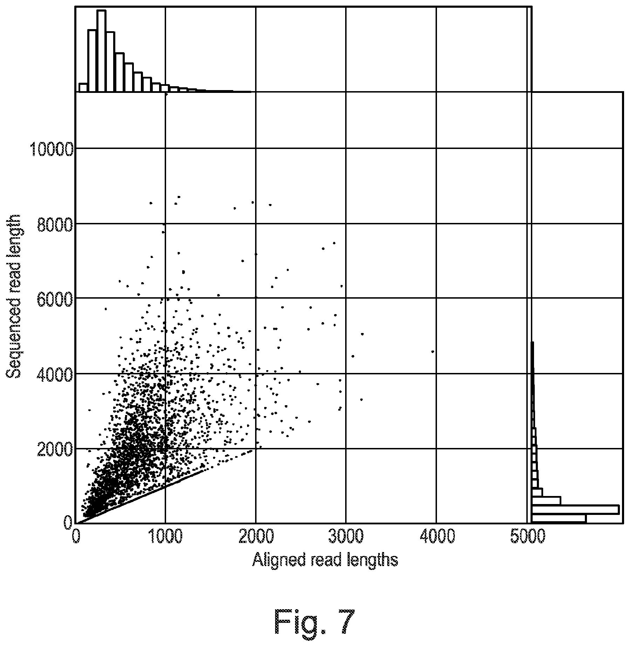

Evidence supporting correct alignment of the individual DNA fragments within each read can be seen in Figure 7. This figure shows the "Sequenced read length" over the "Aligned read length". The sequenced read length is the length of each sequencing read (each read consists of multiple small fragments stuck together). If the short fragments within each sequencing read were aligned to the correct locations on the genome, the "aligned read lengths" would be expected to be shorter than the "sequenced read lengths", which is exactly what is shown in Figure 7. This demonstrates the alignment algorithm is capable of aligning the short fragments within each sequencing read.

To ensure that the correct sites were captured, the sequencing data obtained was compared to MeDIP sequencing data. MedIP is a method which captures methylated sites of the genome, in contrast to the method of the invention which captures unmethylated sites. Therefore, the method of the invention should not be capturing the same locations of the genome as MeDIP.

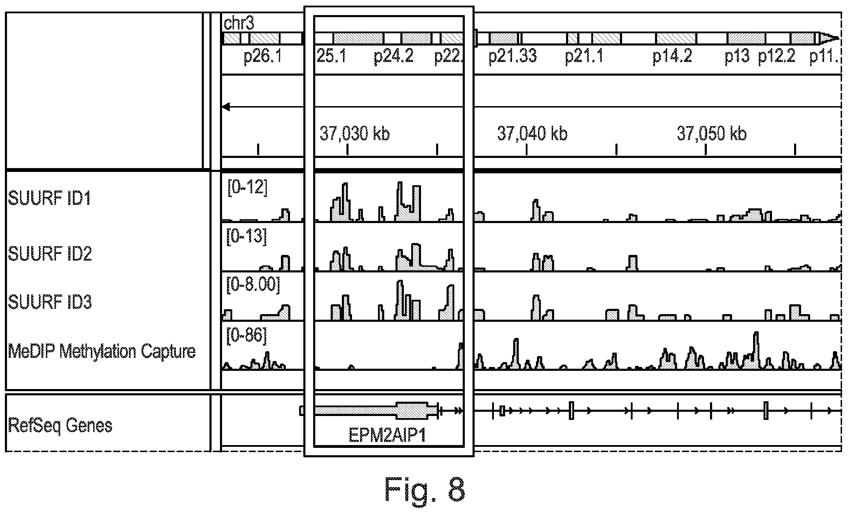

Figure 8 shows a comparison of sequencing reads obtained using the method (SUURF ID1, ID2 and ID3) with a sequencing read obtained using MeDIP. The region shown in the box is a gene promoter sequence which is known to be unmethylated. In this region, it can be seen that there is a build up of sequencing reads from the SUURF samples (capture of non-methylated DNA), and a decrease in sequencing reads from the MedIP sample (capture of methylated DNA). This demonstrates that the method of the invention can be used to successfully capture and sequence non-methylated regions of the genome.

### Example 4: Human head and neck cancer capture and release for illumina sequencing

Head and neck squamous cell carcinoma (HNSCC) (VU40T) DNA was fragmented to 150 bp in the following reaction; 50 µl DNA (5.6 µg), 6.5 µl 10X Fragmentase Reaction Buffer v2, 3 µl NEBNext dsDNA Fragmentase enzyme (M0348), 3.5 µl 200 mM MgCl2, 2 µl nuclease free water to a final volume of 65 µl. The reaction was incubated at 37 °C for 35 mins. To stop the reaction 35 µl 400mM EDTA was added. The DNA was then purified using 2.5 × AMPure XP beads (Beckman), and the size checked using a 1% agarose gel, pre stained with GelRed^{®} (Biotium) (120V for 55 mins).

Next a labelling reaction was prepared containing; 19.4 µl fragmented head and neck cancer DNA (800ng) (150bp), 10 µl M.Mpel enzyme (double mutant (Q136A/N374A)) (stock 1.7 mg/ml), 7 µl 10x CutSmart^{®} Buffer (NEB), 2.33 AdoHcy-8-Hy cofactor (500 µM final) and 31.27 µl nuclease free water to a final volume of 70 µl, the reaction was then incubated at 37 °C for 1h. Next, 4 µl µL proteinase K (800 units/ml) (NEB) was added and the sample was incubated for a further 1h at 50 °C. Finally, 4 µl of Sulfo-DBCO-Biotin Conjugate (15mM stock) (Jena Bioscience) was added and the same was incubated for 1h 37 °C. The sample was then purified using 2.5 × AMPure XP beads (Beckman) and the DNA concentration was quantified using Qubit^{™} 4 Fluorometer and dsDNA HS Assay Kit (Thermo Fisher).

Following this, 600 ng of fragmented and biotinylated DNA in 50 µl Tris buffer A (10 mM Tris, 1 mM Nacl, pH 7.5) was incubated with 60 µl of washed Dynabeads^{™} MyOne^{™} Streptavidin C1 beads (Thermo Fisher) at RT for 20 mins. The beads were then washed 2x with 100 µl Tris buffer A to remove any non-specifically bound DNA. The captured DNA was released from the beads using 90 µl of release buffer (11.2mM ammonium acetate (pH 6.5), 1M NaCl) and 10 ul of 0.85M hydroxylamine solution (170mM final) at 50 °C, 1200 rpm for 1h. The released DNA was then purified using 2.5 × AMPure XP beads (Beckman) and the DNA concentration was quantified using Qubit^{™} 4 Fluorometer and dsDNA HS Assay Kit (Thermo Fisher). The sample was then prepared for illumina sequencing using the KAPA HyperPrep Kit following manufacturer's instructions.

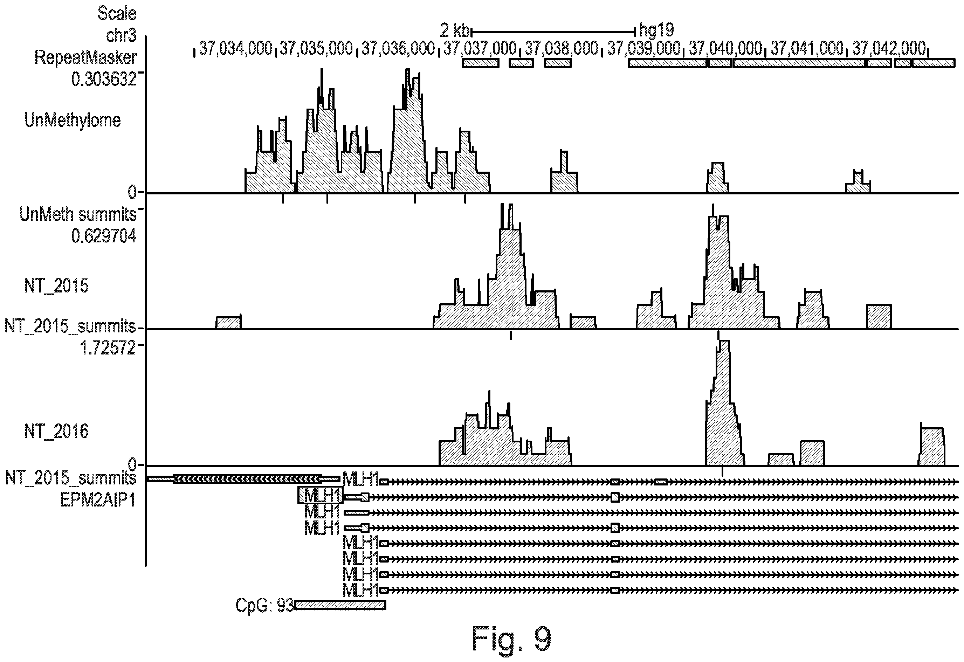

Figure 9 shows a data comparison from the genome browser showing number of reads mapped in a region containing a CpG island at the start of the MLM1 gene for (top) the present unmethylome chemistry showing the location of unmethylated CpG sites and (middle and bottom) MeDIP data (antibody-based capture) showing complementary capture of methylated regions of the genome.

### Example 5: Amplification of captured DNA

### Labelling of unmethylated genomic DNA in fixed cells with methyltransferase enzymes and AdoMet analogue

1×10⁶ cells (MCF7 or MCF10A) were seeded on 10 cm dish and incubated for 24 hrs. Then cells were fixed with 5 ml of cold MeOH/AcOH (95:5) for 10 minutes at -20°C and washed 2x PBS. Fixed cells were incubated for 1 hr at 37°C with 5 ml of the solution in 1x CutSmart buffer:
Taq - 37.4 µl of M.Taql (WT) (1.1 mg/ml), 4 µM AdoHcy-6-N3
M.Mpel - 90.9 µl M.Mpel (double mutant (Q136A/N374A)) (6.9 mg/ml), 65 µM AdoHcy-6-N3 Washed twice with PBS, incubated overnight at RT with 100 µM sulfo-DBCO-Biotin in PBS. Cells were then washed 3x PBST. 1 ml of PBST was added and cells were scraped. DNA was purified using QIAGEN Genomic-tip 20/G. Biotin labelled DNA was then resuspended in 100 mM Tris-HCl pH 8.5 and sonicated to 150 bp.

### Labelling of unmethylated genomic DNA in vitro with methyltransferase enzymes and AdoMet analogue

2 µg of extracted genomic DNA from MCF7 or MCF10A cells was incubated with 0.5 µl of M.Taq (1.1 mg/ml) or M.Mpel (6.9 mg/ml) and AdoHcy-6-N3 (4 µM and 65 µM respectively) in 20 µL total volume of 1x CutSmart buffer for 1 hour at 37°C. 3 µL of Proteinase K was added and samples were incubated for 1 hour at 50°C followed by incubation with 2 mM of DBCO-sulfo-Biotin for 1 hour at 37°C. Biotin labelled DNA was then purified with GenElute Bacterial Genomic DNA Kit. DNA was eluted twice in 10 mM Tris-HCl pH 8.5.

### Library construction (following procedure from Ponnaluri, V.K.C., et al. Genome Biol 18, 122 (2017))

1 µg of DNA was end-repaired, dA-tailed, and ligated with NEBNext Ultra8482 II DNA Library Prep Kit. Without further purification, the ligation product was mixed with 50 µL of Streptavidin magnetic beads (Invitrogen 65001, blocked using 0.1% cold fish gelatin in 1× PBS overnight at 4 °C) in 1 mL of B&W buffer (10 mM Tris-HCl pH 8.0, 1 mM EDTA, 2 M NaCl). Biotin labelled DNA was captured by streptavidin magnetic beads at 4 °C for 2 h with end-over-end rotation. The beads were washed four times with B&W buffer plus 0.05% of Triton X-100 followed by one wash with TE plus Triton X-100. The beads were resuspended in 40 µL of nuclease-free water and 4 µL was used for library amplification using standard PCR. It was found that the presence of the biotin labels did not affect the amplification process.

## Claims

1. A method for analyzing DNA, the method comprising:
- forming labelled DNA fragments by:
(a) cleaving genomic DNA into DNA fragments;
(b) selectively functionalizing any non-methylated CpG sites present in the DNA with a linker comprising a hydrolysable moiety; and
(c) attaching a label to the linker;
- separating the labelled DNA fragments from any non-labelled DNA fragments;
- hydrolysing the hydrolysable moiety of the linker of the separated labelled DNA fragments, so as to release the DNA fragments from the label; and
- sequencing the released DNA fragments.

2. The method of claim 1, wherein step (c) is carried out before step (b), and/or
wherein step (a) is carried out after step (b) or after step (c).

3. The method of claim 1 or claim 2, wherein selectively functionalizing any non-methylated CpG sites in the DNA with the linker is carried out using a DNA methyltransferase enzyme which is capable of selectively transferring a transferable group from a S-adenosyl-L-methionine cofactor analogue to the non-methylated CpG sites of the DNA, wherein the transferrable group constitutes the linker.

4. The method of claim 3, wherein the DNA methyltransferase is a cytosine-5 methyltransferase, optionally wherein the cytosine-5 methyltransferase is M.Mpel, further optionally wherein the DNA methyltransferase is a double mutant (Q136A/N374A) of M.Mpel.

5. The method of any preceding claim, wherein the hydrolysable moiety comprises an imine moiety, an oxime moiety, or a hydrazone moiety, optionally wherein the hydrolysable moiety comprises a Schiff base.

6. The method of any one of claims 3 to 5, wherein the S-adenosyl-L-methionine analogue has the following general formula: 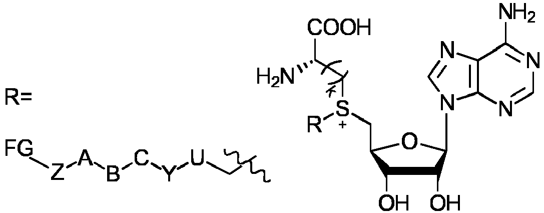
wherein R represents a transferable group, which constitutes the linker;
FG represents a functional group;
Z represents a non-reactive group selected from one of an aliphatic linkage or an aromatic linkage;
A-B-C represent the hydrolysable moiety;
Y represents a non-reactive group selected from one of an aliphatic linkage or an aromatic linkage;
U represents an unsaturated bond, for example selected from one of an alkene, an alkyne, an aryl group, a carbon atom comprising a carbonyl group, a sulphur atom comprising one or two S=O bonds; and
k represents an integer of 1 or 2, optionally wherein:
Z comprises a polyether chain; and/or
is selected from one of an azide, an alkyne, an isothiocyanate, or an isocyanate moiety.

7. The method of claim 6, wherein the S-adenosyl-L-methionine analogue has the following general formula: 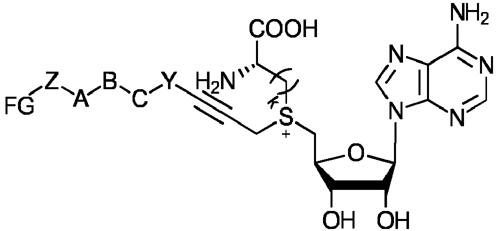 ; or 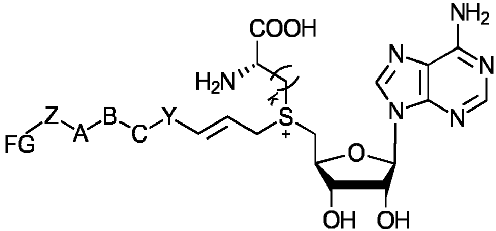 ; or 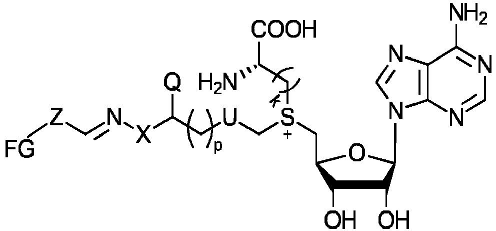
wherein the hydrolysable moiety is a Schiff base moiety comprising the C=N-X-C-Q moiety;
p represents an integer of from 1 to 15;
Q represents one of an oxygen atom or two hydrogen atoms independently bonded to the carbon centre;
X represents one of an oxygen atom or a nitrogen atom;
Z represents a non-reactive group selected from one of an aliphatic linkage or an aromatic linkage;
U represents an unsaturated bond selected from one of an alkene, an alkyne, an aryl group, a carbon atom comprising a carbonyl group, a sulphur atom comprising one or two S=O bonds;
k represents an integer of 1 or 2; and
FG represents the functional group.

8. The method of claim 6 or claim 7, wherein the linker molecule has the following general formula: 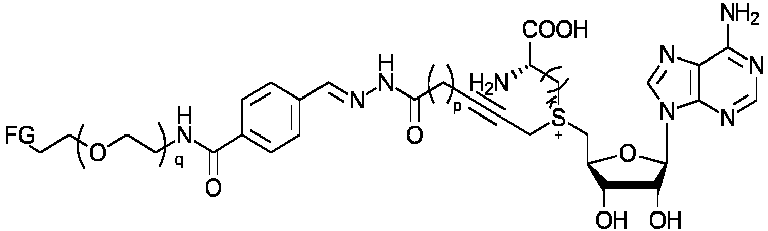
wherein the hydrolysable moiety is -C=N-N-C=O; or 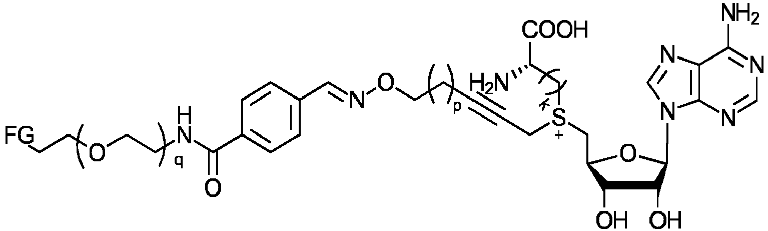
wherein the hydrolysable moiety is the -C=N-O- bond,
wherein p represents an integer of from 1 to 15;
q represents an integer of from 1 to 15;
k represents an integer of 1 or 2; and
FG represents the second functional group.

9. The method of claim 8, wherein FG is an azide moiety, p is 4 and q is 2 or 3, optionally wherein k is 2.

10. The method of any preceding claim, wherein attaching a label to the linker comprises forming a covalent bond between a reactive centre of a functional group of the linker, and the label.

11. The method of any preceding claim, wherein the label comprises a ligand conjugated to a moiety comprising a second functional group which is capable of reacting with the functional group of the linker molecule to form a covalent bond, optionally wherein the label comprises biotin conjugated to a moiety comprising an alkyne.

12. The method of any preceding claim, wherein separating the labelled DNA fragments from any non-labelled DNA fragments comprises using an immobilised capture agent which selectively binds to the label, optionally wherein the capture agent comprises avidin or streptavidin.

13. The method of any preceding claim, further comprising:
ligating the released DNA fragments together, prior to sequencing; and/or
amplifying the DNA prior to sequencing, optionally using PCR amplification.

14. The method of any preceding claim, wherein the DNA is sequenced using nanopore sequencing.

15. The method of any preceding claim, wherein cleavage of the genomic DNA is carried out using a restriction enzyme.

## Patentansprüche

1. Verfahren zum Analysieren von DNA, das Verfahren umfassend:
- Bilden von markierten DNA-Fragmenten durch:
(a) Aufspalten genomischer DNA in DNA-Fragmente,
(b) selektives Funktionalisieren aller nicht-methylierten CpG-Stellen, die in der DNA vorhanden sind, mit einem Linker, der eine hydrolysierbare Einheit umfasst, und
(c) Anbringen einer Markierung an dem Linker,
- Abtrennen der markierten DNA-Fragmente von allen nicht markierten DNA-Fragmenten;
- Hydrolysieren der hydrolysierbaren Einheiten des Linkers der abgetrennten markierten DNA-Fragmente, um die DNA-Fragmente von der Markierung freizusetzen; und
- Sequenzieren der freigesetzten DNA-Fragmente.

2. Verfahren nach Anspruch 1, wobei Schritt (c) vor Schritt (b) durchgeführt wird und/oder wobei Schritt (a) nach Schritt (b) oder nach Schritt (c) durchgeführt wird.

3. Verfahren nach Anspruch 1 oder Anspruch 2, wobei ein selektives Funktionalisieren aller nicht-methylierten CpG-Stellen in der DNA mit dem Linker unter Verwendung eines DNA-Methyltransferase-Enzyms durchgeführt wird, das in der Lage ist, eine übertragbare Gruppe von einem S-Adenosyl-L-Methionin-Cofaktor-Analogon selektiv auf die nicht-methylierten CpG-Stellen der DNA zu übertragen, wobei die übertragbare Gruppe den Linker konstituiert.

4. Verfahren nach Anspruch 3, wobei die DNA-Methyltransferase eine Cytosin-5-Methyltransferase ist, optional, wobei die Cytosin-5-Methyltransferase M.Mpel ist, ferner optional, wobei die DNA-Methyltransferase eine Doppelmutante (Q136A/N374A) von M.Mpel ist.

5. Verfahren nach einem vorherigen Anspruch, wobei die hydrolysierbare Einheit eine Imineinheit, eine Oximeinheit oder eine Hydrazoneinheit umfasst, optional wobei die hydrolysierbare Einheit eine Schiff sche Base umfasst.

6. Verfahren nach einem der Ansprüche 3 bis 5, wobei das 5-Adenosyl-L-Methionin-Analogon die folgende allgemeine Formel aufweist: 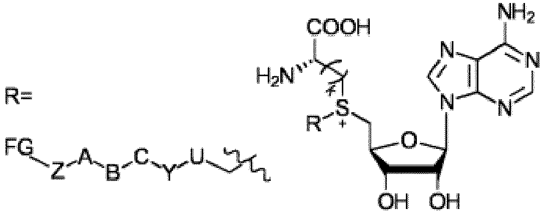
wobei R eine übertragbare Gruppe darstellt, die den Linker konstituiert;
FG eine funktionelle Gruppe darstellt;
Z eine nicht reaktive Gruppe darstellt, ausgewählt aus einer von einer aliphatischen Bindung oder einer aromatischen Bindung;
A-B-C die hydrolysierbare Einheit darstellen;
Y eine nicht reaktive Gruppe darstellt, ausgewählt aus einer von einer aliphatischen Bindung oder einer aromatischen Bindung;
U eine ungesättigte Bindung darstellt, beispielsweise ausgewählt aus einem von einem Alken, einem Alkin, einer Arylgruppe, einem Kohlenstoffatom umfassend eine Carbonylgruppe, einem Schwefelatom umfassend eine oder zwei S=O-Bindungen; und
k eine ganze Zahl von 1 oder 2 darstellt, optional wobei:
Z eine Polyetherkette umfasst, und/oder
ausgewählt ist aus einem Azid, einem Alkin, einem Isothiocyanat oder einer Isocyanateinheit.

7. Verfahren nach Anspruch 6, wobei das 5-Adenosyl-L-Methionin-Analogon die folgende allgemeine Formel aufweist: 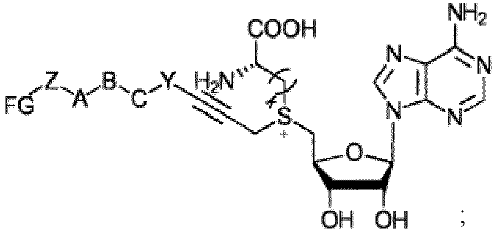 oder 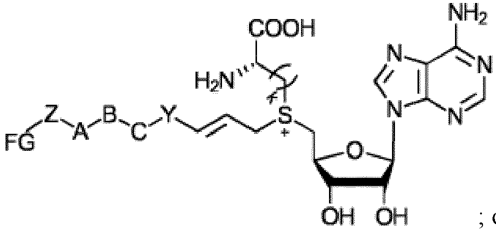 oder 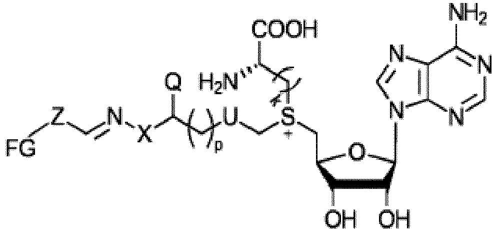
worin die hydrolysierbare Einheit eine Schiff sche Baseneinheit umfassend die C=N-X-C-Q-Einheit ist,
p eine ganze Zahl von 1 bis 15 darstellt;
Q eines von einem Sauerstoffatom oder zwei Wasserstoffatomen darstellt, die unabhängig an das Kohlenstoffzentrum gebunden sind,
X eines von einem Sauerstoffatom oder einem Stickstoffatom darstellt,
Z eine nicht reaktive Gruppe darstellt, ausgewählt aus einer von einer aliphatischen Bindung oder einer aromatischen Bindung;
U eine ungesättigte Bindung darstellt, ausgewählt aus einem von einem Alken, einem Alkin, einer Arylgruppe, einem Kohlenstoffatom umfassend eine Carbonylgruppe, einem Schwefelatom umfassend eine oder zwei S=O-Bindungen;
k eine ganze Zahl von 1 oder 2 darstellt; und
FG die funktionelle Gruppe darstellt.

8. Verfahren nach Anspruch 6 oder Anspruch 7, wobei das Linkermolekül die folgende allgemeine Formel aufweist: 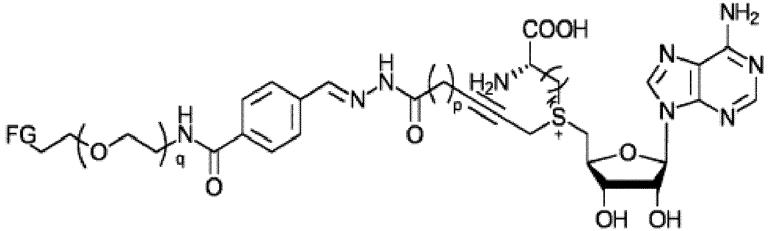
wobei die hydrolysierbare Einheit -C=N-N-C=O ist, oder 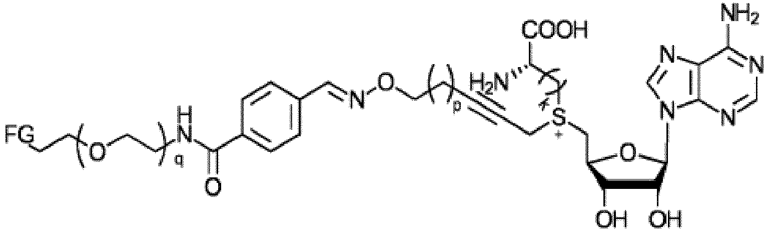
wobei die hydrolysierbare Einheit die -C=N-O-Bindung ist,
wobei p eine ganze Zahl von 1 bis 15 darstellt;
q eine ganze Zahl von 1 bis 15 darstellt;
k eine ganze Zahl von 1 oder 2 darstellt; und
FG die zweite funktionelle Gruppe darstellt.

9. Verfahren nach Anspruch 8, wobei FG eine Azideinheit ist, p 4 ist und q 2 oder 3 ist, optional wobei k 2 ist.

10. Verfahren nach einem vorherigen Anspruch, wobei ein Anbringen einer Markierung an den Linker ein Bilden einer kovalenten Bindung zwischen einem reaktiven Zentrum einer funktionellen Gruppe des Linkers und der Markierung umfasst.

11. Verfahren nach einem vorherigen Anspruch, wobei die Markierung einen Liganden umfasst, der mit einer Einheit konjugiert ist, die eine zweite funktionelle Gruppe umfasst, die in der Lage ist, mit der funktionellen Gruppe des Linkermoleküls zu reagieren, um eine kovalente Bindung zu bilden, optional wobei die Markierung Biotin umfasst, das mit einer Einheit konjugiert ist, die ein Alkin umfasst.

12. Verfahren nach einem vorherigen Anspruch, wobei ein Abtrennen der markierten DNA-Fragmente von allen nicht markierten DNA-Fragmenten ein Verwenden eines immobilisierten Fangmittels umfasst, das selektiv an die Markierung bindet, optional wobei das Fangmittel Avidin oder Streptavidin umfasst.

13. Verfahren nach einem vorherigen Anspruch, ferner umfassend:
Ligieren der zusammen freigesetzten DNA-Fragmente vor einem Sequenzieren, und/oder
Amplifizieren der DNA vor Sequenzieren, optional unter Verwendung von PCR-Amplifikation.

14. Verfahren nach einem vorherigen Anspruch, wobei die DNA unter Verwendung von Nanoporen-Sequenzierung sequenziert wird.

15. Verfahren nach einem vorherigen Anspruch, wobei ein Aufspalten der genomischen DNA unter Verwendung eines Restriktionsenzyms durchgeführt wird.

## Revendications

1. Procédé d'analyse de l'ADN comprenant :
- la formation de fragments d'ADN marqués par :
(a) clivage d'ADN génomique en fragments d'ADN ;
(b) fonctionnalisation sélective de tous les sites CpG non méthylés présents dans l'ADN avec un lieur comprenant une fraction hydrolysable ; et
(c) fixation d'une étiquette au lieur ;
- la séparation des fragments d'ADN étiquetés des fragments d'ADN non étiquetés ;
- l'hydrolyse de la fraction hydrolysable du lieur des fragments d'ADN marqués séparés, de manière à libérer les fragments d'ADN de l'étiquette ; et
- le séquençage des fragments d'ADN libérés.

2. Procédé de la revendication 1, dans lequel l'étape (c) est effectuée avant l'étape (b), et/ou dans lequel l'étape (a) est effectuée après l'étape (b) ou après l'étape (c).

3. Procédé de la revendication 1 ou de la revendication 2, dans lequel la fonctionnalisation sélective de tous les sites CpG non méthylés dans l'ADN avec le lieur est effectuée à l'aide d'une enzyme méthyltransférase d'ADN capable de transférer sélectivement un groupe transférable à partir d'un cofacteur S-adénosyl-L- méthionine analogue aux sites CpG non méthylés de l'ADN, le groupe transférable constituant le lieur.

4. Procédé de la revendication 3, dans lequel la méthyltransférase d'ADN est une cytosine-5 méthyltransférase, la cytosine-5 méthyltransférase étant éventuellement M.Mpel, la méthyltransférase d'ADN étant une double mutante (Q136A/N374A) de M.Mpel.

5. Procédé d'une quelconque revendication précédente, dans lequel la fraction hydrolysable comprend une fraction imine, une fraction oxime ou une fraction hydrazone, la fraction hydrolysable comprenant éventuellement une base de Schiff.

6. Procédé de l'une quelconque des revendications 3 à 5, dans lequel l'analogue de la *S-*adénosyl-L-méthionine a la formule générale suivante : 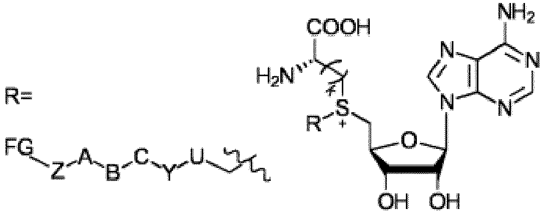
dans laquelle R représente un groupe transférable, qui constitue le lieur ;
FG représente un groupe fonctionnel ;
Z représente un groupe non réactif choisi parmi une liaison aliphatique ou une liaison aromatique ;
A-B-C représente la fraction hydrolysable ;
Y représente un groupe non réactif choisi parmi une liaison aliphatique ou une liaison aromatique ;
U représente une liaison insaturée, par exemple choisie parmi un alcène, un alcyne, un groupe aryle, un atome de carbone comprenant un groupe carbonyle, un atome de soufre comprenant une ou deux liaisons S=O ; et
k représente un entier de 1 ou 2, éventuellement dans laquelle :
Z comprend une chaine polyéther ; et/ou
est choisi parmi un azide, un alcyne, un isothiocyanate ou une fraction isocyanate.

7. Procédé de la revendication 6, dans lequel l'analogue de la 5-adénosyl-L-méthionine a la formule générale suivante : 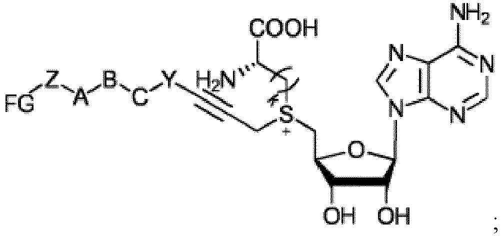 ou 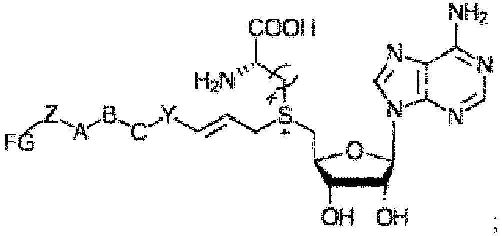 ou 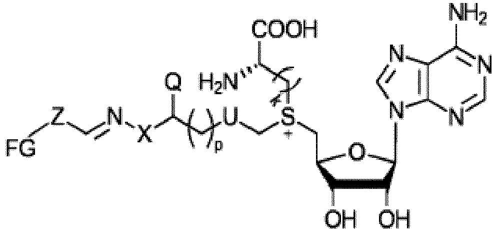
dans laquelle la fraction hydrolysable est une fraction base de Schiff comprenant la fraction C=N-X-C-Q ; p représente un entier de 1 à 15 ;
Q représente un atome d'oxygène ou deux atomes d'hydrogène liés indépendamment au centre carbone ;
X représente un parmi un atome d'oxygène ou un atome d'azote ;
Z représente un groupe non réactif choisi parmi une liaison aliphatique ou une liaison aromatique ;
U représente une liaison insaturée choisie parmi un alcène, un alcyne, un groupe aryle, un atome de carbone comprenant un groupe carbonyle, un atome de soufre comprenant une ou deux liaison S=O ;
k représente un entier de 1 ou 2 ; et
FG représente le groupe fonctionnel.

8. Procédé de la revendication 6 ou de la revendication 7, dans lequel la molécule de lieur a la formule générale suivante : 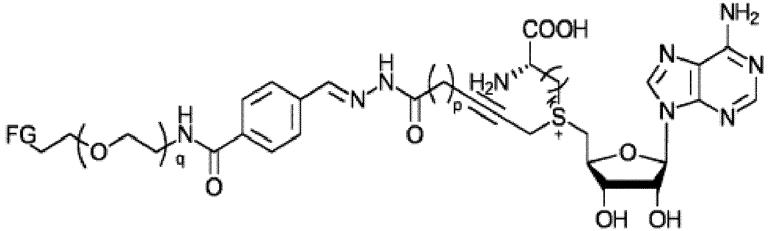
dans laquelle la fraction hydrolysable est -C=N-N-C=O ; ou 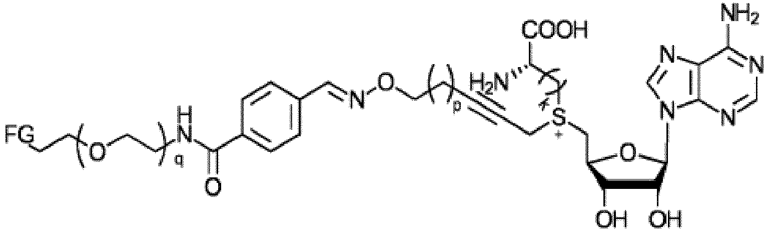
dans laquelle la fraction hydrolysable est la liaison -C=N-O-,
dans laquelle p représente un entier de 1 à 15 ;
q représente un entier de 1 à 15 ;
k représente un entier de 1 ou 2 ; et
FG représente le second groupe fonctionnel.

9. Procédé de la revendication 8, dans lequel FG est une fraction d'azide, p est 4 et q est 2 ou 3, éventuellement dans lequel k est 2.

10. Procédé d'une quelconque revendication précédente, dans lequel la fixation d'une étiquette sur le lieur comprend la formation d'une liaison covalente entre un centre réactif d'un groupe fonctionnel du lieur et l'étiquette.

11. Procédé d'une quelconque revendication précédente, dans lequel l'étiquette comprend un ligand conjugué à une fraction comprenant un second groupe fonctionnel capable de réagir avec le groupe fonctionnel de la molécule de lieur pour former une liaison covalente, éventuellement dans lequel l'étiquette contient de la biotine conjuguée à une fraction comprenant un alcyne.

12. Procédé d'une quelconque revendication précédente, dans lequel la séparation des fragments d'ADN étiquetés des fragments d'ADN non étiquetés comprend l'utilisation d'un agent de capture immobilisé qui se lie sélectivement à l'étiquette, éventuellement dans lequel l'agent de capture comprend l'avidine ou la streptavidine.

13. Procédé d'une quelconque revendication précédente, comprenant en outre :
la ligation des fragments d'ADN libérés ensemble, avant le séquençage ; et/ou
l'amplification de l'ADN avant le séquençage, éventuellement en utilisant l'amplification par PCR.

14. Procédé d'une quelconque revendication précédente, dans lequel l'ADN est séquencé en utilisant le séquençage nanopore.

15. Procédé d'une quelconque revendication précédente, dans lequel le clivage de l'ADN génomique est effectué à l'aide d'une enzyme de restriction.
